# EUROPEAN PATENT APPLICATION

(11) **EP 3 733 840 A1**
(43) Date of publication of application: **04.11.2020**
(21) Application number: 18897015.6
(22) Date of filing: 27.12.2018
(51) Int. Cl.: C12N 5/10, C12N 1/00, C12N 5/0735

(54) **PLURIPOTENT STEM CELL AGGREGATION INHIBITOR**

(30) Priority: 28.12.2017 JP 2017254829
(71) Applicant: Kaneka Corporation, Osaka-shi, Osaka 530-8288 (JP)
(72) Inventor: IBUKI Masato, Kobe-shi, Hyogo 650-0047 (JP)
(74) Representative: Hoffmann Eitle
(86) International application number: PCT/JP2018/048312
(87) International publication number: WO 2019/131940

(57) **Abstract**

The present application provides: a suppressor of pluripotent stem cell aggregation for use in suspension culture of pluripotent stem cells, comprising a cell cycle arresting agent; a method for producing a pluripotent stem cell aggregate(s), comprising a step of subjecting pluripotent stem cells to suspension culture in a culture medium comprising the suppressor; a pluripotent stem cell aggregate obtained by the method; and a composition for culture of pluripotent stem cells comprising a cell aggregate(s) of pluripotent stem cells, a culture medium, and a cell cycle arresting agent. According to the method, it is possible, in suspension culture of pluripotent stem cells, to produce a cell aggregate(s) with high cell viability and appropriate size in a high yield while maintaining the undifferentiated state of the cells and suppressing the aggregation.

SEQUENCE LISTING

<110>  Kaneka Corporation

<120>  Agent for inhibiting aggregation of pluripotent stem cells

<130>  B170690

<150>  JP 2017-254829
<151>  2017-12-28

<160>  4

<170>  PatentIn version 3.5

<210>  1
<211>  28
<212>  PRT
<213>  Artificial

<220>
<223>  synthetic peptide

<400>  1

Arg Lys Lys Arg Arg Gln Arg Arg Arg Gly Pro Pro Val Ile Ala Pro
1               5                   10                  15

Arg Pro Glu His Thr Lys Ser Val Tyr Thr Arg Ser
            20                  25

<210>  2
<211>  6
<212>  PRT
<213>  Artificial

<220>
<223>  synthetic peptide

<220>
<221>  MOD_RES
<222>  (1)..(1)
<223>  ACETYLATION

<220>
<221>  MOD_RES
<222>  (2)..(2)
<223>  PHOSPHORYLATION

<400>  2

Pro Tyr Leu Lys Thr Lys
1               5

<210>  3
<211>  6
<212>  PRT
<213>  Artificial

<220>
<223>  synthetic peptide

<220>
<221>  MOD_RES
<222>  (2)..(2)
<223>  PHOSPHORYLATION

<400>  3

Pro Tyr Leu Lys Thr Lys
1               5

<210>  4
<211>  25
<212>  PRT
<213>  Artificial

<220>
<223>  synthetic peptide

<220>
<221>  DISULFID
<222>  (12)..(12)

<220>
<221>  DISULFID
<222>  (18)..(18)

<220>
<221>  MOD_RES
<222>  (24)..(24)
<223>  PHOSPHORYLATION

<400>  4

Tyr Gly Arg Lys Lys Arg Arg Gln Arg Gly Phe Cys Asp Gly Phe Tyr
1               5                   10                  15

Ala Cys Tyr Lys Asp Val Pro Tyr Leu
            20                  25

## Description

### TECHNICAL FIELD

The present invention relates to a suppressor of pluripotent stem cell aggregation, and to a method for suppressing aggregation of pluripotent stem cells. The present invention also relates to a method for producing a pluripotent stem cell aggregate, and to a pluripotent stem cell aggregate produced by the method. The present invention further relates to a composition for culturing pluripotent stem cells.

### BACKGROUND ART

Recent research on human pluripotent stem cells (e.g., human ES cells and human iPS cells) has increasingly made regenerative medicine come in reality. Because these cells possess an ability to proliferate infinitely and an ability to differentiate into various types of cells, the regenerative medicine using the pluripotent stem cells is expected to radically change therapies against, for example, refractory diseases and lifestyle-related diseases. It has already been possible to induce *in vitro* the differentiation of the pluripotent stem cells into various types of somatic cells, such as neurons, cardiomyocytes, renal cells, hepatocytes, blood cells, retinal cells, and progenitor cells thereof.

Meanwhile, in the practical use of the regenerative medicine in which pluripotent stem cells are used to regenerate a variety of organs, there are strong demands for efficient methods for production of a large number of pluripotent stem cells necessary for regenerating organs or tissues *in vivo.* For example, the regeneration of the liver requires approximately 2 x 10¹¹ cells and, for culturing this number of cells by, for example, adhesion culture on a dish surface, dishes of 10⁶ cm² or more are required, which correspond to approximately 20,000 dishes in case of a typical 10 cm dish. Thus, the scaling up is difficult because the number of cells to be obtained using adhesion culture on a dish surface depends on the culture area, making it difficult to supply a sufficient amount of pluripotent stem cells required for regenerative medicine.

In such situations, researching and developing mass production techniques for pluripotent stem cells by three-dimensional culture, such as suspension culture, instead of adhesion culture have been advanced.

For example, Non-Patent Literature 1 discloses a method for producing spheroids with a uniform size, comprising culturing human pluripotent stem cells in suspension while strongly stirring a liquid culture medium, by using a spinner flask as a cell culture vessel for suspension culture.

Non-Patent Literature 2 discloses a method for producing spheroids with a uniform size in each micro-well,, using a substrate plate on which small micro-wells are formed.

Non-Patent Literature 3 discloses a method of culturing using a culture medium, in which the viscosity and specific gravity are adjusted, while keeping pluripotent stem cells in the state of suspension and suppressing collisions between the cells.

Patent Literature 1 discloses a technology in which cells are cultured with gyratory (or rotation) culture in a liquid culture medium, so that cell aggregates are produced.

Patent Literature 2 discloses a method for subjecting pluripotent stem cells to suspension culture until the average diameter of cell aggregates reaches approximately 200 µm or more and 300 µm or less.

Patent Literature 3 discloses a technique for suppressing cell aggregation by culturing cells in suspension in a culture medium containing lysophospholipids such as lysophosphatidic acid (LPA) and sphingosine-1-phosphate (SIP).

Patent Literature 4 discloses a technique for subjecting human pluripotent stem cells to suspension culture to form cell aggregates in a method for producing retinal cells or retinal tissues.

Patent Literature 5 discloses a technique for the structure of a polymer compound having anionic functional groups (i.e., deacylated gellan gum) capable of uniformly dispersing cells, such as pluripotent stem cells, or tissues in the state of suspension.

### CITATION LIST

### Patent Literature

Patent Literature 1: JP Patent Publication (Kokai) No. 2003-304866A
Patent Literature 2: WO2013/077423A
Patent Literature 3: WO2016/121737A
Patent Literature 4: WO2016/063986A
Patent Literature 5: WO2014/017513A

### Non-Patent Literature

Non-Patent Literature 1: Olmer R. et al., Tissue Engineering: Part C, Volume 18 (10): 772-784 (2012)
Non-Patent Literature 2: Ungrin MD et al., PLoS ONE, 2008, 3(2), e1565 (2008)
Non-Patent Literature 3: Otsuji GT et al., Stem Cell Reports, Volume 2: 734-745 (2014)

### SUMMARY OF INVENTION

### Problem to be Solved by the Invention

The present inventors have found that the non-specific adsorption of membrane protein and cell membrane between cells and the adhesion between cells mediated by cadherin on the cell surface are important mechanisms in culturing adhesive cells, such as pluripotent stem cells, in suspension. That is, a challenge that needs to be addressed in the technique of suspension culture is to produce cell aggregates with an appropriate size without damaging the binding of membrane proteins of the cells, the binding of cadherins on the cell surface, or the like. However, the suspension culture techniques disclosed in Non-Patent Literatures 1 to 3 and Patent Literatures 1, 2, 4 and 5 have the following problems.

The method of Non-Patent Literature 1 likely causes cells to die due to shear stress, which is a defect of the method.

In the method of Non-Patent Literature 2, it is difficult to scale up the method and to exchange a culture medium.

In the method of Non-Patent Literature 3, because of less movement of a culture medium during culture, oxygen and nutritional components are less likely to be supplied to cell aggregates.

Patent Literature 1 fails to disclose a means for controlling the size of cell aggregates to an appropriate size.

Patent Literature 2 discloses adding, to a culture medium, an aqueous polymer as a means for preventing adhesion between cell aggregates, so that the viscosity increases. This causes the same defect as in the case of Non-Patent Literature 3, in which oxygen and nutritional components are less likely to be supplied to cell aggregates.

Patent Literature 4 fails to provide a technique for controlling the size of cell aggregates.

Patent Literature 5 requires the separation of cells from polymer compound structures after culture, because the addition of the structures to the culture medium is needed.

In order to solve the above problems, the present inventors have developed a technique for suspension culture that can suppress the aggregation of cells by subjecting the cells to suspension culture in a culture medium containing lysophospholipid in Patent Literature 3 and thereby to produce cell aggregates with an appropriate size without damaging the cells.

However, Patent Literature 3 discloses only the lysophospholipid as the component that suppresses cell aggregation. The present inventors have contemplated that the culture medium in suspension culture provided with components that suppress or promote cell aggregation, apart from lysophospholipids, could allow the size of cell aggregates to appropriately regulate without relying on mechanical/physical means.

### Means for Solution of Problem

As a result of extensive researches, the present inventors have found that a cell cycle arresting agent exhibits the action of suppressing aggregation of cells by being present in a culture medium in suspension culture, and have completed the present invention.

Accordingly, the present invention encompasses the features listed below.
(1) A suppressor of pluripotent stem cell aggregation for use in suspension culture of pluripotent stem cells, comprising a cell cycle arresting agent.
(2) The suppressor of pluripotent stem cell aggregation according to (1) above, wherein a concentration of the cell cycle arresting agent is 55.0 mg/mL or more and 1.1 g/mL or less, or 60 mM or more and 15 M or less.
(3) The suppressor of pluripotent stem cell aggregation of (1) or (2) above, wherein the cell cycle arresting agent is a concentrated form capable of being diluted, upon culture, to a concentration of 3 ng/mL or more and 30 mg/mL or less, or 3 nM or more and 300 mM or less, in an culture medium.
(4) The suppressor of pluripotent stem cell aggregation according to any one of (1) to (3) above, wherein the cell cycle arresting agent is at least one substance selected from G1 phase-arresting agents, G1/S phase-arresting agents, G2 phase-arresting agents, G2/M phase-arresting agents, and M phase-arresting agents.
(5) The suppressor of pluripotent stem cell aggregation according to any one of (1) to (4) above, wherein the cell cycle arresting agent is at least one substance selected from the group consisting of colchicine, dimethylsulfoxide (DMSO), cytochalasin D, cytochalasin B, docetaxel, podophyllotoxin, demecolcine, nocodazole, jasplakinolide, vinblastine, griseofulvin, vinorelbine, latorunculin A, 2,3,5-triiodobenzoic acid (TIBA), ansamitocin P-3, cytochalasin A, cytochalasin E, indanocine, latorunculin B, wiskostatin, and salts thereof.
(6) The suppressor of pluripotent stem cell aggregation according to (4) above, wherein the G1 phase-arresting agent is at least one substance selected from colchicine and dimethylsulfoxide (DMSO).
(7) The suppressor of pluripotent stem cell according to (4) above, wherein the G1/S phase-arresting agent is cytochalasin D.
(8) The suppressor of pluripotent stem cell aggregation according to (4) above, wherein the G2 phase-arresting agent is at least one substance selected from cytochalasin D, colchicine, and docetaxel.
(9) The suppressor of pluripotent stem cell aggregation according to (4) above, wherein the G2/M phase-arresting agent is at least one substance selected from podophyllotoxin, demecolcine, latorunculin A, cytochalasin B, and nocodazole.
(10) The suppressor of pluripotent stem cell aggregation according to (4) above, wherein the M phase-arresting agent is jasplakinolide.
(11) A method for producing pluripotent stem cell aggregates, comprising a step of subjecting pluripotent stem cells to suspension culture in a culture medium comprising a cell cycle arresting agent.
(12) The method according to (11) above, wherein a concentration of the cell cycle arresting agent is 3 ng/mL or more and 30 mg/mL or less, or 3 nM or more and 300 mM or less, in the culture medium.
(13) The method according to (11) or (12) above, wherein the cell cycle arresting agent is at least one substance selected from G1 phase-arresting agents, G1/S phase-arresting agents, G2 phase-arresting agents, G2/M phase-arresting agents, and M phase-arresting agents.
(14) The method according to any one of (11) to (13) above, wherein the cell cycle arresting agent is at least one substance selected from the group consisting of colchicine, dimethylsulfoxide (DMSO), cytochalasin D, cytochalasin B, docetaxel, podophyllotoxin, demecolcine, nocodazole, jasplakinolide, vinblastine, griseofulvin, vinorelbine, latorunculin A, 2,3,5-triiodobenzoic acid (TIBA), ansamitocin P-3, cytochalasin A, cytochalasin E, indanocine, latorunculin B, wiskostatin, and salts thereof.
(15) The method according to (13) above, wherein the G1 phase-arresting agent is at least one substance selected from colchicine and dimethylsulfoxide (DMSO).
(16) The method according to (13) above, wherein the G1/S phase-arresting agent is cytochalasin D.
(17) The method according to (13) above, wherein the G2 phase-arresting agent is at least one substance selected from cytochalasin D, colchicine, and docetaxel.
(18) The method according to (13) above, wherein the G2/M phase-arresting agent is at least one substance selected from podophyllotoxin, demecolcine, latorunculin A, cytochalasin B, and nocodazole.
(19) The method according to (13) above, wherein the M phase-arresting agent is jasplakinolide.
(20) A pluripotent stem cell aggregate obtained by the method according to any one of (11) to (19) above.
(21) A composition for culture of pluripotent stem cells comprising a cell aggregate(s) of pluripotent stem cells, a culture medium, and a cell cycle arresting agent, wherein a concentration of the cell cycle arresting agent is 3 ng/mL or more and 30 mg/mL or less, or 3 nM or more and 300 mM or less.
(22) The composition for culture of pluripotent stem cells according to (21) above, wherein the cell cycle arresting agent is at least one substance selected from G1 phase-arresting agents, G1/S phase-arresting agents, G2 phase-arresting agents, G2/M phase-arresting agents, and M phase-arresting agents.
(23) The composition for culture of pluripotent stem cells according to (21) or (22) above, wherein the cell cycle arresting agent is at least one substance selected from the group consisting of colchicine, dimethylsulfoxide (DMSO), cytochalasin D, cytochalasin B, docetaxel, podophyllotoxin, demecolcine, nocodazole, jasplakinolide, vinblastine, griseofulvin, vinorelbine, latorunculin A, 2,3,5-triiodobenzoic acid (TIBA), ansamitocin P-3, cytochalasin A, cytochalasin E, indanocine, latorunculin B, wiskostatin, and salts thereof.
(24) The composition for culture of pluripotent stem cells according to any one of (21) to (23) above, further comprising a growth factor.
(25) A method for suppressing aggregation of pluripotent stem cells, comprising a step of subjecting the pluripotent stem cells to suspension culture in a culture medium comprising a cell cycle arresting agent.
(26) The method according to (25) above, wherein a concentration of the cell cycle arresting agent is 3 ng/mL or more and 30 mg/mL or less, or 3 nM or more and 300 mM or less, in the culture medium.
(27) The method according to (25) or (26) above, wherein the cell cycle arresting agent is at least one substance selected from G1 phase-arresting agents, G1/S phase-arresting agents, G2 phase-arresting agents, G2/M phase-arresting agents, and M phase-arresting agents.
(28) The method according to any one of (25) to (27) above, wherein the cell cycle arresting agent is at least one substance selected from the group consisting of colchicine, dimethylsulfoxide (DMSO), cytochalasin D, cytochalasin B, docetaxel, podophyllotoxin, demecolcine, nocodazole, jasplakinolide, vinblastine, griseofulvin, vinorelbine, latorunculin A, 2,3,5-triiodobenzoic acid (TIBA), ansamitocin P-3, cytochalasin A, cytochalasin E, indanocine, latorunculin B, wiskostatin, and salts thereof.
(29) The method according to (27) above, wherein the G1 phase-arresting agent is at least one substance selected from colchicine and dimethylsulfoxide (DMSO).
(30) The method according to (27) above, wherein the G1/S phase-arresting agent is cytochalasin D.
(31) The method according to (27) above, wherein the G2 phase-arresting agent is at least one substance selected from cytochalasin D, colchicine, and docetaxel.
(32) The method according to (27), wherein the G2/M phase-arresting agent is at least one substance selected from podophyllotoxin, demecolcine, latorunculin A, cytochalasin B, and nocodazole.
(33) The method according to (27) above, wherein the M phase-arresting agent is jasplakinolide.
(34) A pluripotent stem cell culture medium comprising a culture medium, and a cell cycle arresting agent, wherein a concentration of the cell cycle arresting agent is 3 ng/mL or more and 30 mg/mL or less, or 3 nM or more and 300 mM or less.
(35) The pluripotent stem cell culture medium according to (34) above, wherein the cell cycle arresting agent is at least one substance selected from G1 phase-arresting agents, G1/S phase-arresting agents, G2 phase-arresting agents, G2/M phase-arresting agents, and M phase-arresting agents.
(36) The pluripotent stem cell culture medium according to (34) or (35) above, wherein the cell cycle arresting agent is at least one substance selected from the group consisting of colchicine, dimethylsulfoxide (DMSO), cytochalasin D, cytochalasin B, docetaxel, podophyllotoxin, demecolcine, nocodazole, jasplakinolide, vinblastine, griseofulvin, vinorelbine, latorunculin A, 2,3,5-triiodobenzoic acid (TIBA), ansamitocin P-3, cytochalasin A, cytochalasin E, indanocine, latorunculin B, wiskostatin, and salts thereof.
(37) The pluripotent stem cell culture medium according to (35) above, wherein the G1 phase-arresting agent is at least one substance selected from colchicine and dimethylsulfoxide (DMSO).
(38) The pluripotent stem cell culture medium according to (35) above, wherein the G1/S phase-arresting agent is cytochalasin D.
(39) The pluripotent stem cell culture medium according to (35) above, wherein the G2 phase-arresting agent is at least one substance selected from cytochalasin D, colchicine, and docetaxel.
(40) The pluripotent stem cell culture medium according to (35) above, wherein the G2/M phase-arresting agent is at least one substance selected from podophyllotoxin, demecolcine, latorunculin A, cytochalasin B, and nocodazole.
(41) The pluripotent stem cell culture medium according to (35) above, wherein the M phase-arresting agent is jasplakinolide.
(42) The pluripotent stem cell culture medium according to any one of (34) to (41) above, further comprising a growth factor.
(43) The pluripotent stem cell culture medium according to any one of (34) to (42) above for producing a cell aggregate.
(44) The method according to any one of (11) to (19) above, the pluripotent stem cell aggregate according to (20) above, the composition for culture of pluripotent stem cells according to any one of (21) to (24) above, or the pluripotent stem cell culture medium according to (43) above, wherein the size of the widest portion of the cell aggregate in 70% or more (by weight) of the cell aggregates is 500 µm or less, preferably 300 µm or less.
(45) The method according to any one of (11) to (19) above and (44) above, the pluripotent stem cell aggregate according to (20) or (44) above, the composition for culture of pluripotent stem cells according to any one of (21) to (24) above and (44) above, or the pluripotent stem cell culture medium according to (43) or (44) above, wherein the size of the widest portion of the cell aggregate in 70% or more (by weight) of the cell aggregates is 40 µm or more, preferably 100 µm or more.

The present specification includes disclosure of JP Patent Application No. 2017-254829, from which the present application claims priority.

### Effects of Invention

In one embodiment, the suppressor of pluripotent stem cell aggregation (active ingredient; at least one cell cycle arresting agent) of the present invention may be added to a culture medium in order to suppress aggregation of pluripotent stem cells during suspension culture.

According to one embodiment of the method for suppressing pluripotent stem cell aggregation of the present invention, the aggregation of pluripotent stem cells may be suppressed in suspension culture in the presence of a suppressor of pluripotent stem cell aggregation as described above.

According to one embodiment of the method for producing a cell aggregate(s) of the present invention, the cell aggregate(s) can be produced in a high yield.

According to one embodiment of the cell aggregate of the present invention, the cell aggregate(s) have an appropriate size and a high viable cell rate.

One embodiment of the cell culture composition of the present invention may be used to produce a cell aggregate(s) in a high yield.

### BRIEF DESCRIPTION OF THE DRAWINGS

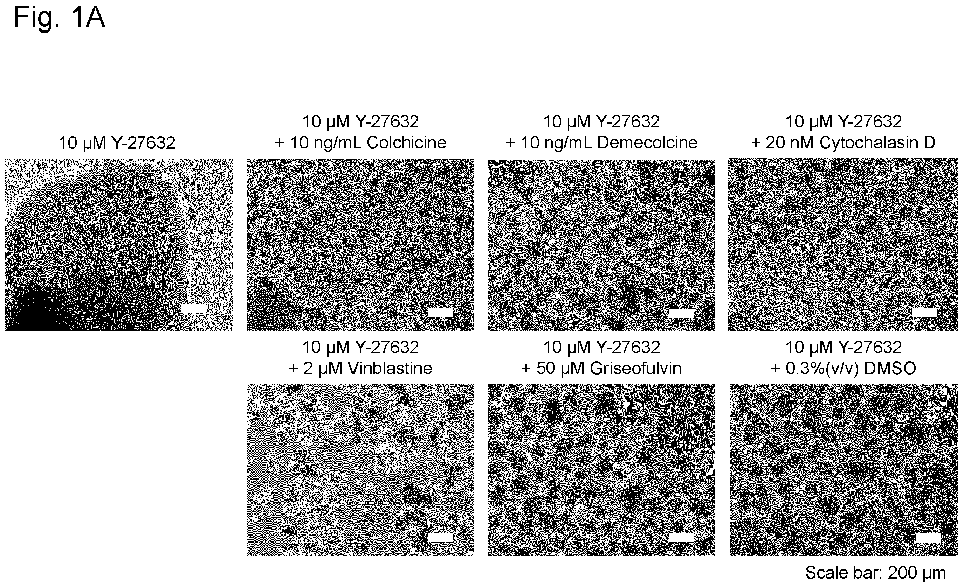

[Fig. 1A] This figure shows the images observed by phase contrast microscopy on Day 1 of culture when human iPS cells were subjected to suspension culture in the presence of Y-27632 (10 µM) in a culture medium comprising a cell cycle arresting agent, i.e., colchicine, demecolcine, cytochalasin D, vinblastine, griseofulvin, or dimethylsulfoxide (DMSO) at the concentration indicated in the figure. The control is the image observed by phase contrast microscopy on Day 1 of culture when human iPS cells were subjected to suspension culture in the presence of Y-27632 (10 µM) in a culture medium free of the cell cycle arresting agent. The scale bar on each image datum is 200 µm.

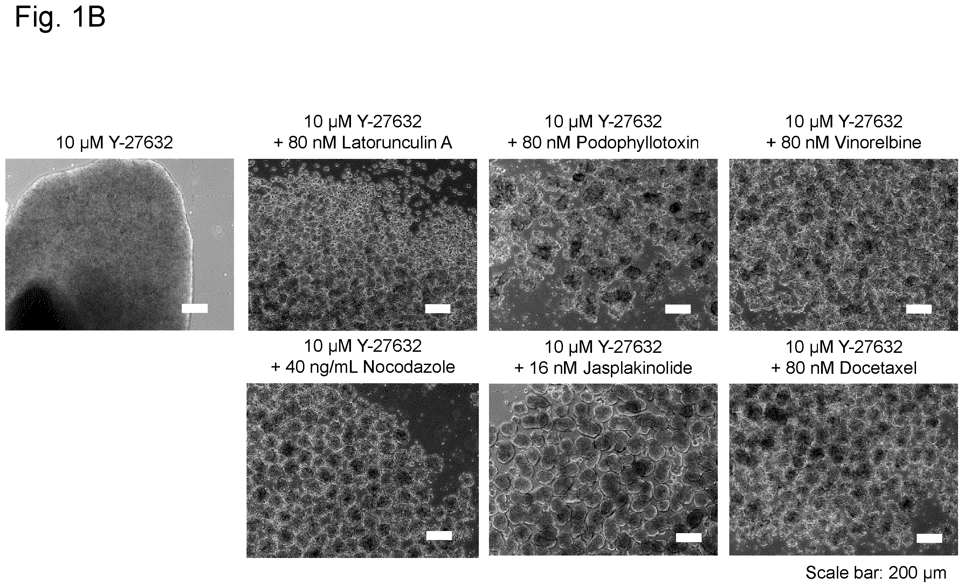

[Fig. 1B] This figure shows the images observed by phase contrast microscopy on Day 1 of culture when human iPS cells were subjected to suspension culture in the presence of Y-27632 (10 µM) in a culture medium comprising a cell cycle arresting agent, i.e., latorunculin A, podophyllotoxin, vinorelbine, nocodazole, jasplakinolide, or docetaxel, at the concentration indicated in the figure. The control is the image observed by phase contrast microscopy on Day 1 of culture when human iPS cells were subjected to suspension culture in the presence of Y-27632 (10 µM) in a culture medium free of the cell cycle arresting agent. The scale bar on each image datum is 200 µm.

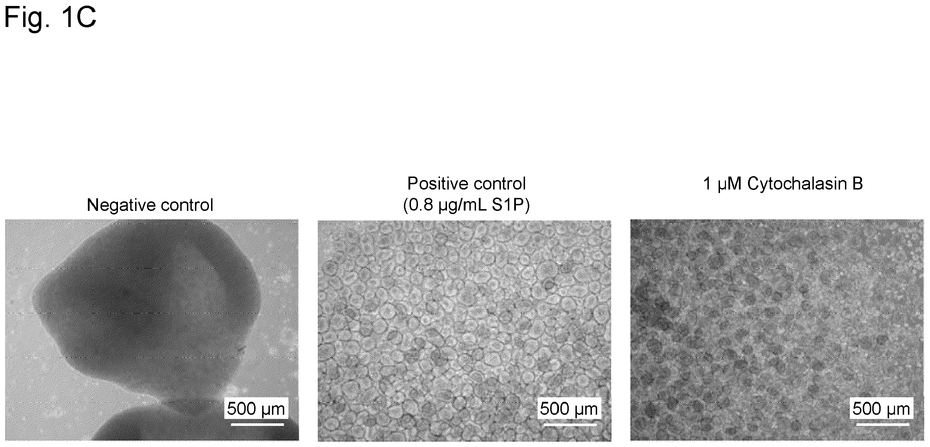

[Fig. 1C] This figure shows the images observed by phase contrast microscopy on Day 1 of culture when human iPS cells were subjected to suspension culture in the presence of Y-27632 (10 µM) in a culture medium free of cell cycle arresting agents (negative control), in a culture medium comprising sphingosine-1-phosphate (SIP) (0.8 µg/mL (2.1 µM)) (positive control), or in a culture medium comprising cytochalasin B (1 µM). The scale bar on each image datum is 500 µm.

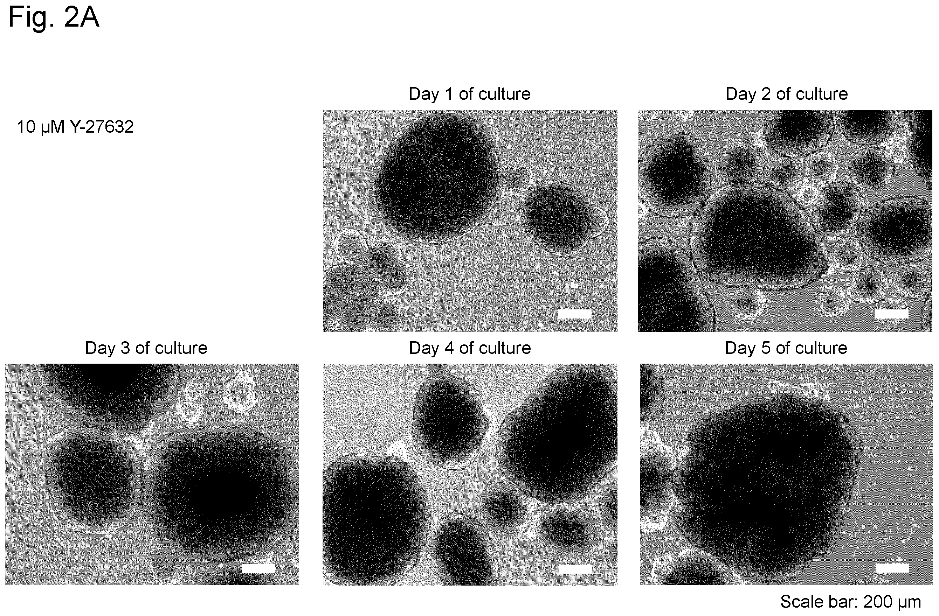

[Fig. 2A] This figure shows the images observed by phase contrast microscopy from Day 1 to Day 5 of culture when human iPS cells were subjected to suspension culture in a culture medium comprising Y-27632 (10 µM) to produce cell aggregates and then continuously subjected to suspension culture. The scale bar on each image datum is 200 µm.

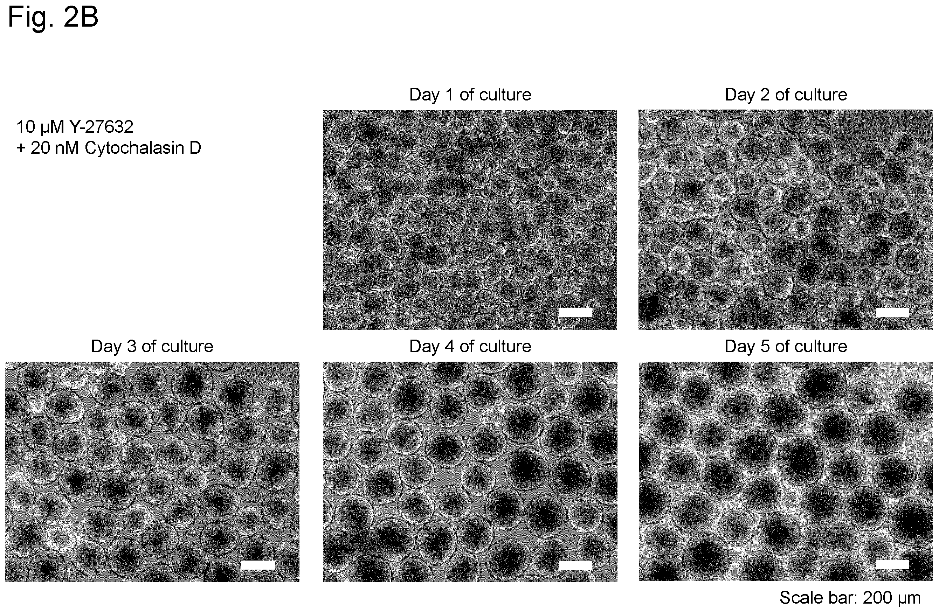

[Fig. 2B] This figure shows the images observed by phase contrast microscopy from Day 1 to Day 5 of culture when human iPS cells were subjected to suspension culture in the presence of Y-27632 (10 µM) in a culture medium comprising cytochalasin D (20 nM) to produce cell aggregates and then continuously subjected to suspension culture. The scale bar on each image datum is 200 µm.

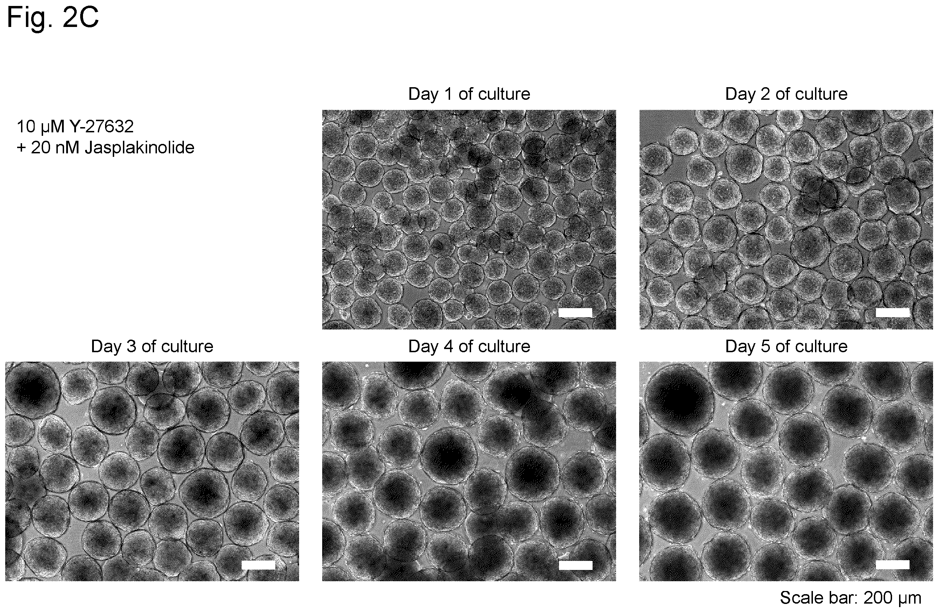

[Fig. 2C] This figure shows the images observed by phase contrast microscopy from Day 1 to Day 5 of culture when human iPS cells were subjected to suspension culture in the presence of Y-27632 (10 µM) in a culture medium comprising jasplakinolide (20 nM) to produce cell aggregates and then continuously subjected to suspension culture. The scale bar on each image datum is 200 µm.

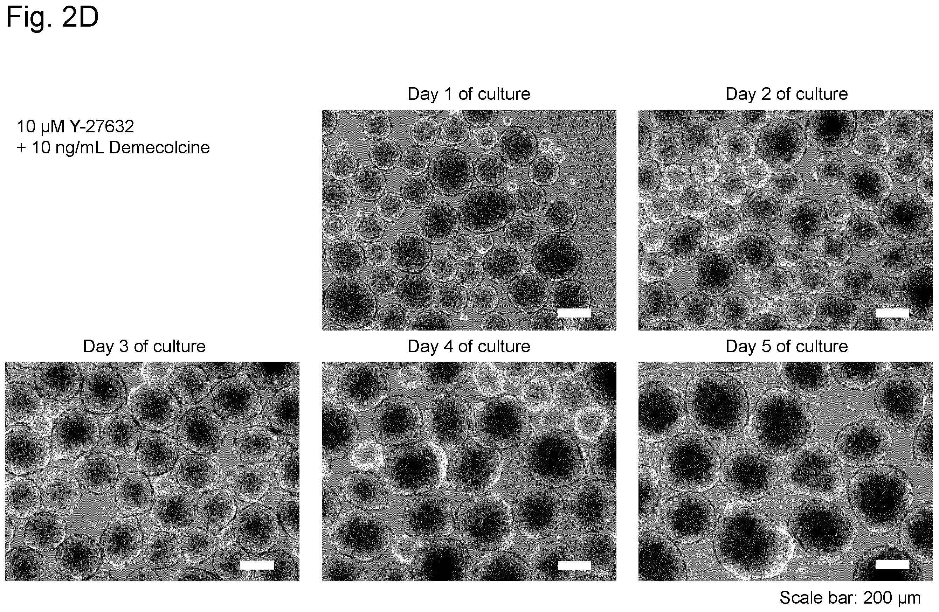

[Fig. 2D] This figure shows the images observed by phase contrast microscopy from Day 1 to Day 5 of culture when human iPS cells were subjected to suspension culture in the presence of Y-27632 (10 µM) in a culture medium comprising demecolcine (10 ng/mL (26.9 nM)) to produce cell aggregates and then continuously subjected to suspension culture. The scale bar on each image datum is 200 µm.

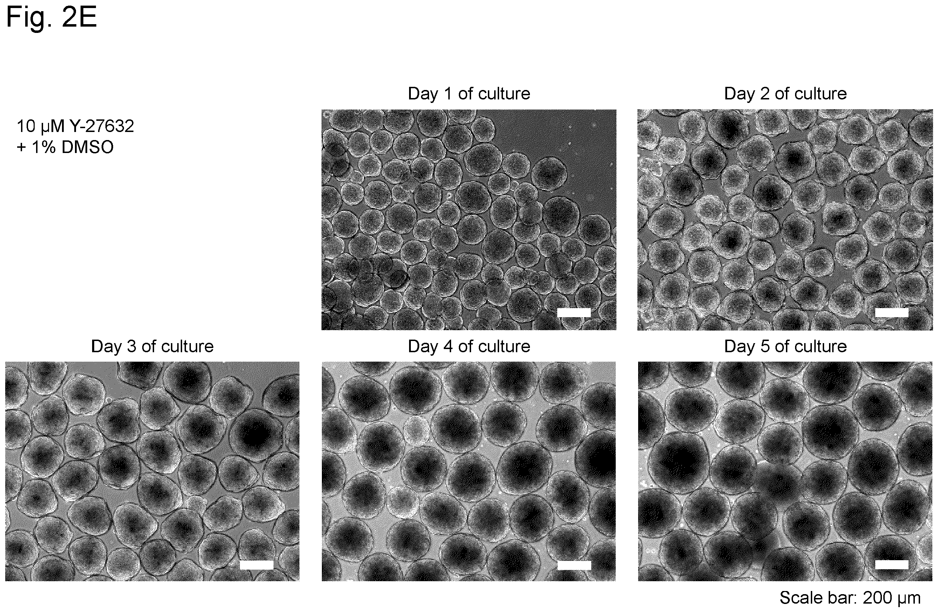

[Fig. 2E] This figure shows the images observed by phase contrast microscopy from Day 1 to Day 5 of culture when human iPS cells were subjected to suspension culture in the presence of Y-27632 (10 µM) in a culture medium comprising DMSO (1% (0.14 M)) to produce cell aggregates and then continuously subjected to suspension culture. The scale bar on each image datum is 200 µm.

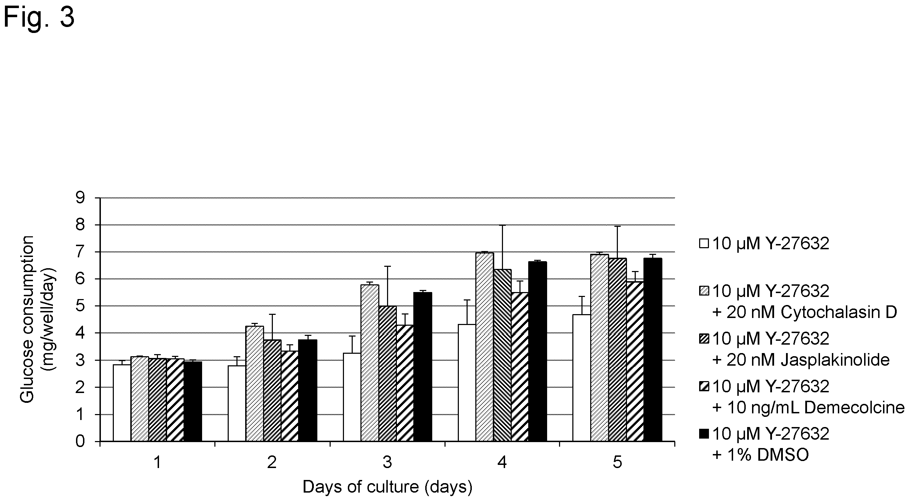

[Fig. 3] This figure is a graph showing glucose consumption from Day 1 to Day 5 of culture when human iPS cells were subjected to suspension culture in the presence of Y-27632 (10 µM) in a culture medium comprising cytochalasin D (20 nM), jasplakinolide (20 nM), demecolcine (10 ng/mL (26.9 nM), or DMSO (1% (0.14 M)) to produce cell aggregates and then continuously subjected to suspension culture. The control is glucose consumption when cultured in a culture medium comprising Y-27632 (10 µM).

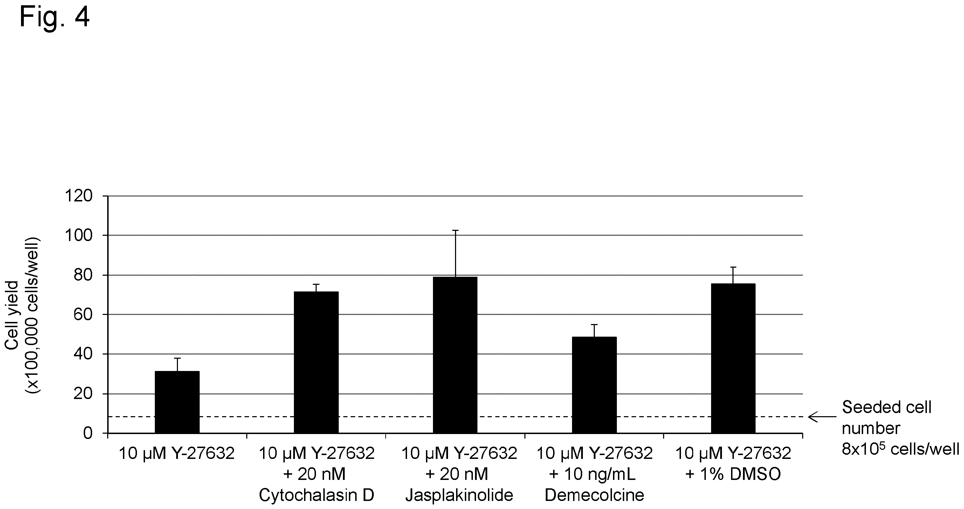

[Fig. 4] This figure is a graph showing cell yields on Day 5 of culture when human iPS cells (seeded cell number, 8 x 10⁵ cells/well) were subjected to suspension culture in the presence of Y-27632 (10 µM) in a culture medium comprising cytochalasin D (20 nM), jasplakinolide (20 nM), demecolcine (10 ng/mL (26.9 nM)), or DMSO (1% (0.14 M)) to produce cell aggregates and then continuously subjected to suspension culture. The control is a cell yield when cultured in a culture medium comprising Y-27632 (10 µM).

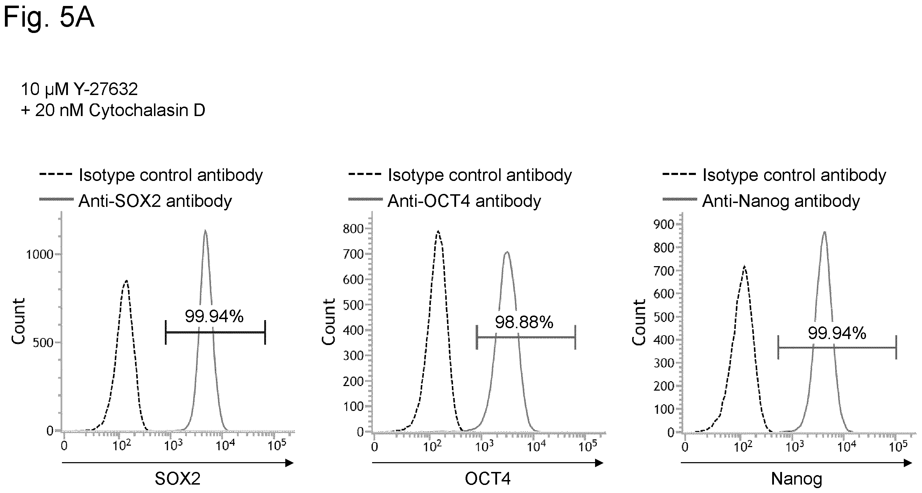

[Fig. 5A] This figure shows the results measured for the percentage of cells on Day 5 of culture that were positive for undifferentiation markers (SOX2, OCT4, and Nanog) when human iPS cells (seeded cell number, 8 x 10⁵ cells/well) were subjected to suspension culture in the presence of Y-27632 (10 µM) in a culture medium comprising cytochalasin D (20 nM).

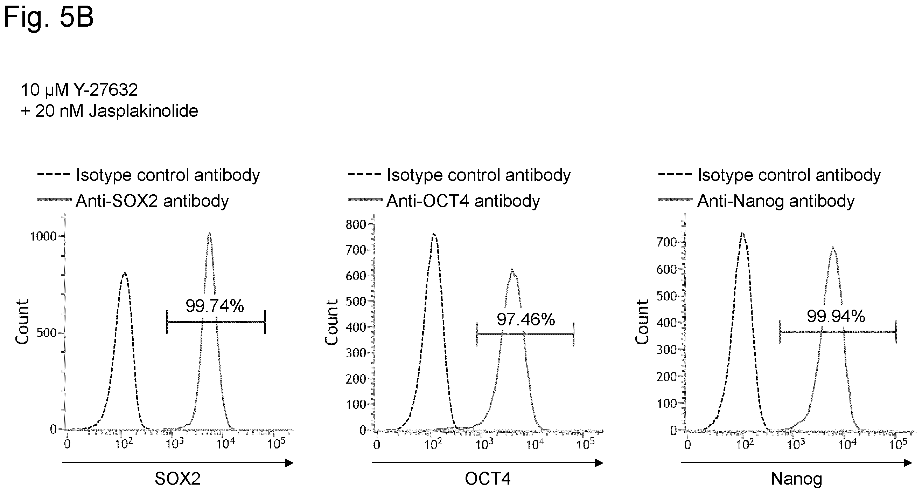

[Fig. 5B] This figure shows the results measured for the percentage of cells on Day 5 of culture that were positive for undifferentiation markers (SOX2, OCT4, and Nanog), when human iPS cells (seeded cell number, 8 x 10⁵ cells/well) were subjected to suspension culture in the presence of Y-27632 (10 µM) in a culture medium comprising jasplakinolide (20 nM).

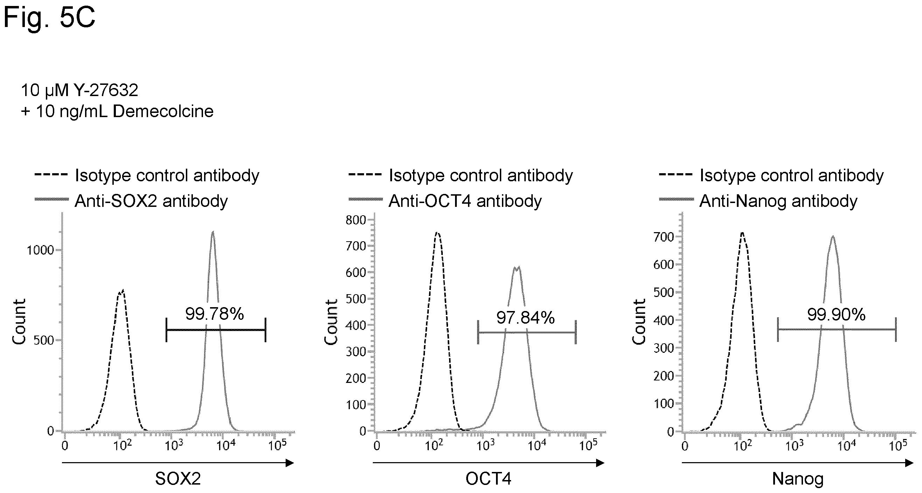

[Fig. 5C] This figure shows the results measured for the percentage of cells on Day 5 of culture that were positive for undifferentiation markers (SOX2, OCT4, and Nanog) when human iPS cells (seeded cell number, 8 x 10⁵ cells/well) were subjected to suspension culture in the presence of Y-27632 (10 µM) in a culture medium comprising demecolcine (10 ng/mL (26.9 nM)).

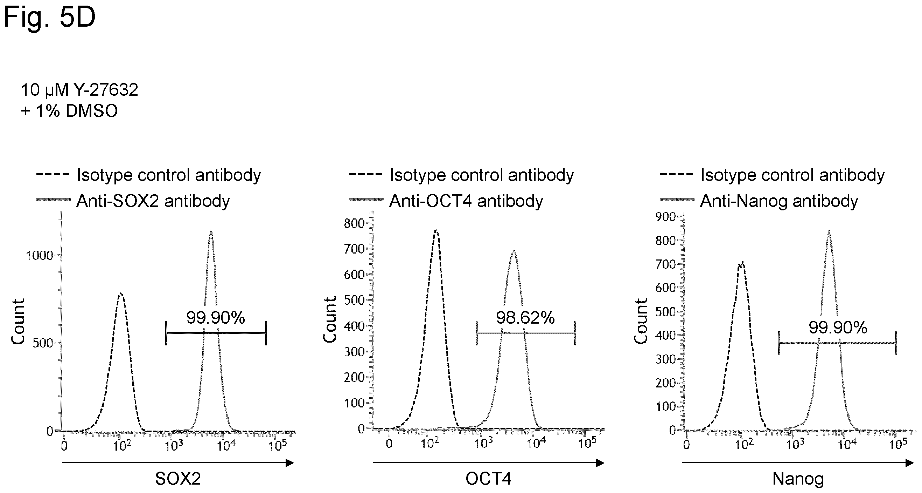

[Fig. 5D] This figure shows the results measured for the percentage of cells on Day 5 of culture that were positive for undifferentiation markers (SOX2, OCT4, and Nanog) when human iPS cells (seeded cell number, 8 x 10⁵ cells/well) were subjected to suspension culture in the presence of Y-27632 (10 µM) in a culture medium comprising DMSO (1% (0.14 M)).

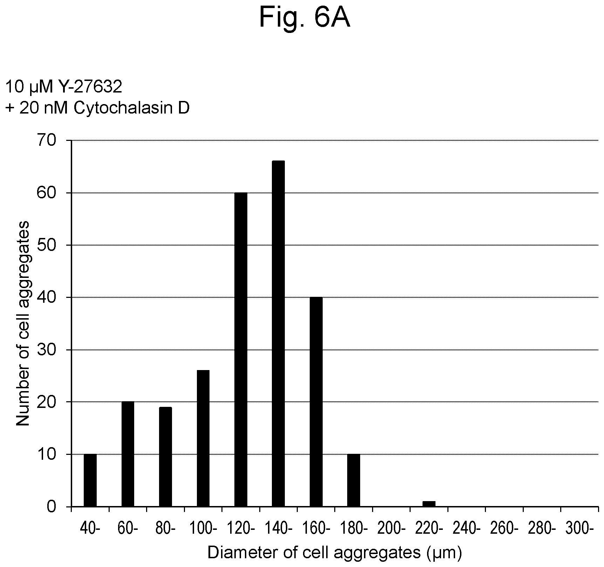

[Fig. 6A] This figure is the graph showing the distribution of diameters of cell aggregates on Day 1 of culture when human iPS cells were subjected to suspension culture in the presence of Y-27632 (10 µM) in a culture medium comprising cytochalasin D (20 nM) to produce cell aggregates.

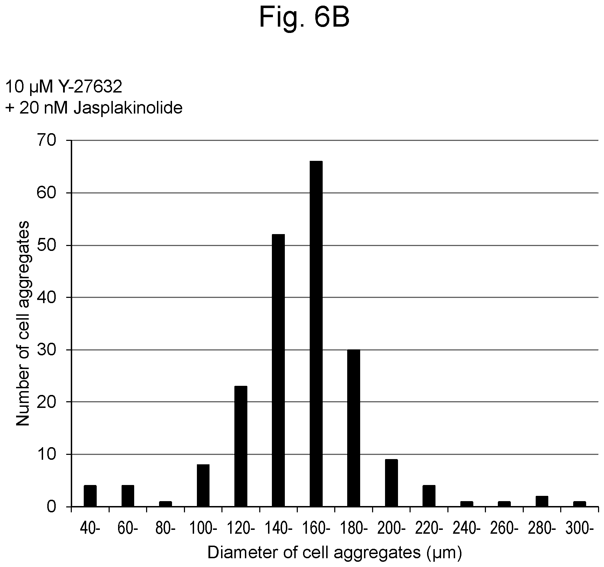

[Fig. 6B] This figure is the graph showing the distribution of diameters of cell aggregates on Day 1 of culture when human iPS cells were subjected to suspension culture in the presence of Y-27632 (10 µM) in a culture medium comprising jasplakinolide (20 nM) to produce cell aggregates.

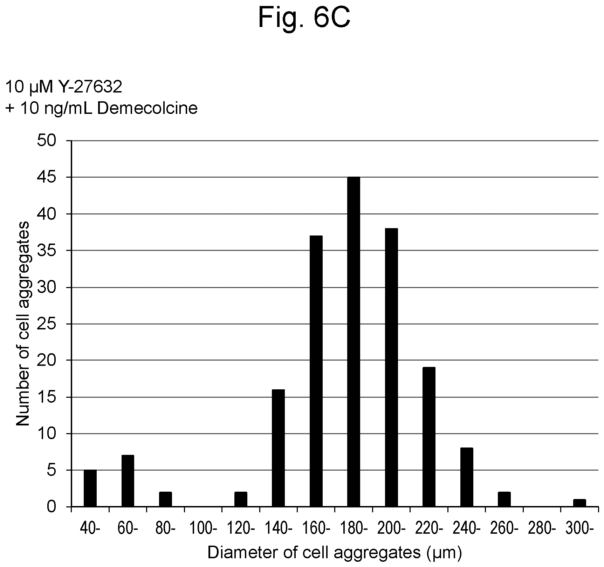

[Fig. 6C] This figure is the graph showing the distribution of diameters of cell aggregates on Day 1 of culture when human iPS cells were subjected to suspension culture in the presence of Y-27632 (10 µM) in a culture medium comprising demecolcine (10 ng/mL (26.9 nM)) to produce cell aggregates.

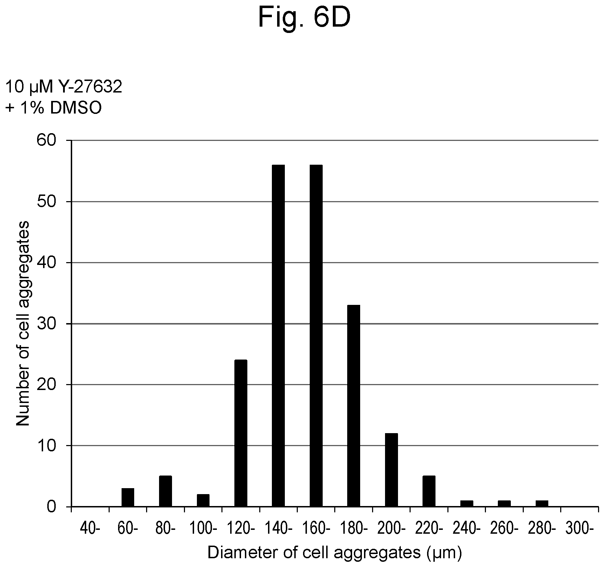

[Fig. 6D] This figure is the graph showing the distribution of diameters of cell aggregates on Day 1 of culture when human iPS cells were subjected to suspension culture in the presence of Y-27632 (10 µM) in a culture medium comprising DMSO (1% (0.14 M)) to produce cell aggregates.

### DESCRIPTION OF EMBODIMENTS

Hereinafter, referring to preferable embodiments, the present invention will be described in detail.

### <1. Pluripotent Stem Cells>

The pluripotent stem cell used in the present invention has a property of forming an embryoid body or cell assemblies (hereinafter, collectively referred to as "cell aggregate") in a culture medium. Specific examples of such cells can include animal-derived pluripotent stem cells, preferably mammal-derived pluripotent stem cells, more preferably human-derived pluripotent stem cells.

As used herein, the "pluripotent stem cell" refers to a cell that has both pluripotency and high self-replication ability. The pluripotency is the ability to differentiate into all three germ layers (i.e. endoderm, mesoderm, and ectoderm) and the high self-replication ability is the ability to proliferate infinitely.

Specific examples of the pluripotent stem cells include, but are not limited to, embryonic stem cells (ES cells), EG cells, which are pluripotent stem cells derived from fetal primordial germ cells, (Shamblott M. J. et al., Proc. Natl. Acad. Sci. USA. 95: 13726-13731 (1998)), GS cells, which are testis-derived pluripotent stem cells, (Conrad S., Nature, 456: 344-349 (2008)), and iPS cells (induced pluripotent stem cells), which are somatic cell-derived artificial pluripotent stem cells. Preferred pluripotent stem cells are ES cells and iPS cells.

ES cells are pluripotent stem cells derived from early embryos called blastocysts. iPS cells are artificially established pluripotent stem cells to which pluripotency has been conferred by introducing reprogramming factors into a somatic cell so that the somatic cell is reprogrammed into the undifferentiated state. Examples of the reprogramming factors that can be used include a combination of OCT3/4 and KLF4, SOX2 and c-Myc (or L-Myc) (Takahashi K, et al. Cell. 131: 861-872 (2007)), and a combination of OCT3/4, SOX2, LIN28 and Nanog (Yu J, et al. Science. 318: 1917-1920 (2007)). Examples of ways for introducing these factors into a cell include, but are not particularly limited to, a gene transfer with vectors such as viruses and plasmids, synthetic RNA introduction, and a direct injection of protein. In addition, iPS cells may be used the cells that are created using, for example, microRNA, RNA, small molecule compounds and the like.

As the pluripotent stem cells such as ES cells and iPS cells, commercially available cells or assigned cells may be used, or newly generated cells may be used. For example, known cell lines, including 253G1, 201B6, 201B7, 409B2, 454E2, HiPS-RIKEN-1A, HiPS-RIKEN-2A, HiPS-RIKEN-12A, Nips-B2, TkDN4-M, TkDA3-1, TkDA3-2, TkDA3-4, TkDA3-5, TkDA3-9, TkDA3-20, hiPSC 38-2, MSC-iPSC1, BJ-iPSC1 iPSC1, and RPChiPS771-2 cell lines, may be used as the iPS cells. As the ES cells, known ES cell lines that may be used include KhES-1, KhES-2, KhES-3, KhES-4, KhES-5, SEES-1, SEES-2, SEES-3, SEES-4, SEES-5, SEES-6, SEES-7, HUES8, CyT49, H1, H9, and HS-181. Newly generated clinical-grade iPS or ES cells may also be used. Examples of cell sources when iPS cells are generated include, but are not particularly limited to, fibroblasts and lymphocytes.

The pluripotent stem cells used in the present invention may be derived from any animal, examples of which may include: mammals such as rodents (e.g., mouse, rat, and hamster), primates (e.g., human, gorilla, and chimpanzee), and domestic animals and pets (e.g., dog, cat, rabbit, cow, horse, pig, sheep, and goat). Particularly preferred are cells derived from humans.

Pluripotent stem cells proliferated and produced by culture, such as adhesion culture, while maintaining a property of undifferentiation by known methods in order to be subjected to suspension culture of the present invention, need to separate the cells from vessels or single-cellulize, for example, using detaching agents, because the cells are attached or adhesive to each other and to the vessels. Methods of the separation are not particularly limited, and examples of the detaching agent include enzymes, such as trypsin or collagenase, and a mixture of the enzyme with a chelating agent such as EDTA (ethylene diamine tetraacetic acid). Examples of enzymes as the detaching agents include, but are not particularly limited to, trypsin, Accutase (registered trade mark; Thermo Fisher Scientific Co., Ltd.), TrypLE™ Express Enzyme (Life Technologies Japan Ltd.), TrypLE™ Select Enzyme (Life Technologies Japan Ltd.), Dispase (registered trade mark), and collagenase. The separated pluripotent stem cells or cell population thereof may be cryopreserved as needed and thawed to use a predetermined amount when subjected to suspension culture for cell proliferation according to the present invention.

### <2. Cell Aggregate>

As used herein, the "cell aggregate" refers to a cell population being in the state of cell assemblies, formed by a plurality of cells aggregated three-dimensionally (also referred to as a "spheroid"). Cell aggregates typically have a substantially spherical shape. The aggregation of identical cells includes the aggregation in which an aggregate is formed by the proliferation of a single cell. The mechanism of cell aggregation includes, but is not limited to, for example, non-specific adsorption of membrane proteins and cell membranes between cells, and adhesion between cells mediated by cadherin on the cell surface.

In the present invention, pluripotent stem cells that constitute a cell aggregate may be composed of a single cell line or may be composed of multiple cell lines, preferably composed of a single cell line. Alternatively, a cell aggregate composed of pluripotent stem cells, such as human induced pluripotent stem cells (iPS cells) or human embryonic stem cells (ES cells), includes the cells that express pluripotent stem cell markers and/or are positive for pluripotent stem cell markers. Herein, the pluripotent stem cell markers are substantially common to iPS cells and ES cells. Examples of the pluripotent stem cell makers include alkaline phosphatase, NANOG, OCT4, SOX2, TRA-1-60, c-Myc, KLF4, LIN28, SSEA-4, and SSEA-1. Such pluripotent stem cell markers can be used as indicators for proliferation and management of cells while ensuring that the undifferentiated state of the cells can be maintained.

The pluripotent stem cell markers can be detected by any detection method in the art. Examples of the method of detecting cell markers include, but are not limited to, flow cytometry. In the flow cytometry using a fluorescently labeled antibody as a detection reagent, when cells emitting stronger fluorescence compared to negative control (isotype control) are detected, the cells are determined to be "positive" for the marker. The percentage of cells positive for fluorescently labeled antibodies analyzed by flow cytometry is sometimes referred to as a "positive rate". As the fluorescently labeled antibodies, any antibody known in the art can be used, and examples of the antibodies include, but are not limited to, antibodies labeled with fluorescein isothiocyanate (FITC), phycoerythrin (PE), allophycocyanin (APC), or the like.

The positive rate of the pluripotent stem cell marker according to the detection method described above may be preferably 80% or more, more preferably 90% or more, more preferably 91% or more, more preferably 92% or more, more preferably 93% or more, more preferably 94% or more, more preferably 95% or more, more preferably 96% or more, more preferably 97% or more, more preferably 98% or more, more preferably 99% or more, and more preferably 100% or less. Here, the pluripotent stem cell markers are synonymous with undifferentiation markers, and the both can be used interchangeably.

In the present invention, cell aggregation of pluripotent stem cells is suppressed in suspension culture by the presence of a cell cycle arresting agent in the culture medium.

As used herein, the term "suppression of cell aggregation", "suppressing cell aggregation", "suppressing aggregation of cells", or a similar term thereof refers to suppressing cell aggregation, thereby suppressing the formation or increase of cell aggregates. As used herein, the term "suppressor of cell aggregation" refers to a substance or agent that has an effect of suppressing cell aggregation, and in the present invention, it is a composition comprising a cell cycle arresting agent, e.g., a substance or agent that regulates cytoskeletons to arrest the cell cycle.

The size of the cell aggregate produced by the method of the present invention is not particularly limited, and when observed under a microscope, the upper limit of the size of the widest portion in observed images is, for example, 900 µm or less, 800 µm or less, 700 µm or less, 600 µm or less, 500 µm or less, 400 µm or less, or 300 µm or less. The lower limit is, for example, 40 µm or more, 50 µm or more, 60 µm or more, 70 µm or more, 80 µm or more, 90 µm or more, or 100 µm or more. Generally, the size of a single human iPS cell is approximately 10 µm. The cell aggregates with such a size range have a preferable environment for cell proliferation because oxygen and nutritional components are easily supplied to their inner cells.

It is preferable in a cell aggregate population produced by the method of the present invention that, for example, 70% or more, 80% or more, 90% or more, or 95% or more by weight of the cell aggregates constituting the cell aggregate population have a size within the above-mentioned range.

### <3. Culture and Culture Medium>

As used herein, the term "suspension culture" refers to a method of cell culture in which cells are proliferated in a suspension state in a culture medium. In the suspension culture according to the method of the present invention, the size of the cell aggregate of pluripotent stem cells that is formed in the culture solution added with a suppressor of cell aggregation comprising a cell cycle arresting agent, is suppressively controlled to the predetermined size.

The "suspension culture method" is a method for suggesting cells to suspension culture, wherein the cells in the method are present as a mass of cells aggregated in the culture solution. The "suspension culture method" is unlimitedly used herein as a method for culturing cells three-dimensionally for mass production. Another culture method other than the suspension culture method is an adhesion culture method. The "adhesion culture method" is a method for suggesting cells to adhesion culture. The "adhesion culture" refers to make cells adhered to an external matrix such as a culture vessel or the like to proliferate principally in a monolayer. In the present invention, the culture method for producing cell aggregates and controlling their size is a method for suspension culture. However, culture methods used in the stages other than main step, such as those used in the maintenance culture stage are not limited to suspension culture methods. Here, the aforementioned adhesive cells can generally be cultured not only in adhesion culture, but also in suspension culture.

As used herein, the "culture medium" refers to a liquid or solid substance prepared for culturing cells. In principle, the "culture medium" contains at least minimum requirement of components essential for proliferation and/or maintenance of cells. The culture medium used herein is, unless otherwise stated, a liquid culture medium for animal cells used in culture of animal-derived cells.

Seed cells of pluripotent stem cells subjected to suspension culture culture can be obtained by culture such as adhesion culture described in <1. Pluripotent Stem Cells> above. For example, a predetermined cell line can be proliferated by adhesion culture to obtain a predetermined cell number of cell lines. The "adhesion culture" generally refers to culturing cells on the surface of a culture vessel or a polymer support, or on feeder cells (e.g., mouse fibroblasts) to allow the cells to proliferate principally in the form of a monolayer. The culture medium for proliferation of pluripotent stem cells by suspension culture needs to select those suitable for maintaining the cell quality (e.g., undifferentiation) that are susceptible to the culture environment. It is preferable to use a serum-free culture medium in order to maintain the stability of the quality and to eliminate harmful substances such as pathogens. Various serum-free synthetic media are commercially available or described in literatures or the like. Such culture media can be used in the culture of the present invention (e.g., Mika Kan and Miho Furue, Bioengineering, vol. 92, pp. 487-490, 2014 (The Society for Biotechnology, Japan); R. Ian Freshney, Culture of Animal Cells: A Manual of Basic Technique and Specialized Applications (Sixth Ed.), John Wiley&Sons, Inc., 2010). The culture medium used in the present invention is preferably a culture medium suitable for suspension culture of pluripotent stem cells, typically a liquid medium comprising a basal medium for animal cells and an additive(s).

As used herein, the "basal medium" refers to a medium that is the base of a variety of culture media for animal cells. Culture is possible with the basal medium alone, but the basal medium can also be used with a variety of culture additives to prepare culture media specific for different cells according to purposes. Examples of the basal medium that can be used herein include, but are not particularly limited to, BME medium, BGJb medium, CMRL1066 medium, Glasgow MEM medium, Improved MEM Zinc Option medium, IMDM medium (Iscove's Modified Dulbecco's Medium), Medium 199 medium, Eagle MEM medium, αMEM medium, DMEM medium (Dulbecco's Modified Eagle's Medium), Ham's F10 medium, Ham's F12 medium, RPMI 1640 medium, Fischer's medium, and a mixed medium thereof (e.g., DMEM/F12 medium (Dulbecco's Modified Eagle's Medium/Nutrient Mixture F-12 Ham)). The DMEM/F12 medium is used, in particular, by mixing DMEM medium and Ham's F12 medium in a ratio of preferably 60/40 to 40/60 by weight, more preferably 55/45 to 45/55 by weight, and most preferably 50/50 by weight (at equivalent amount).

The culture medium used in the present invention is preferably a medium containing no serum (a serum-free medium) or a medium containing serum replacement. Examples of the serum replacement include KnockOut™ Serum Replacement (KSR) (Gibco).

Additives in the culture medium used in the present invention are not limited, and can include, for example, the components listed below.

As the additives, it is preferable to include at least one selected from L-ascorbic acid, insulin, transferrin, selenium, and sodium bicarbonate, and more preferably include all of these. The L-ascorbic acid, insulin, transferrin, selenium, and sodium bicarbonate may be added to the medium in the form of, for example, a solution, derivative, salt, or mixed reagent. For example, L-ascorbic acid may be added to the medium in the form of a derivative such as magnesium-ascorbyl-2-phosphate. Selenium may be added to the medium in the form of a selenite (e.g., sodium selenite). The insulin and transferrin may be natural products isolated from a tissue or serum of an animal (e.g., preferably a human, mouse, rat, cow, horse, goat). They may be genetically engineered recombinant proteins. The insulin, transferrin, and selenium may be added to the medium in the form of a reagent ITS (insulin-transferrin-selenium). The ITS is a cell growth-promoting additive containing insulin, transferrin, and sodium selenite.

As a medium comprising the additives described above, for example, a commercially available culture medium comprising at least one selected from L-ascorbic acid, insulin, transferrin, selenium, and sodium bicarbonate may be used. Examples of a commercially available culture medium supplemented with insulin and transferrin may include CHO-S-SFM II (Life Technologies Japan Ltd.), Hybridoma-SFM (Life Technologies Japan Ltd.), eRDF Dry Powdered Media (Life Technologies Japan Ltd.), UltraCULTURE™ (BioWhittaker, Inc.), UltraDOMA™ (BioWhittaker, Inc.), UltraCHO™ (BioWhittaker, Inc.), and UltraMDCK™ (BioWhittaker, Inc.). For, example, STEMPRO™ hESC SFM (Life Technologies Japan Ltd.), mTeSR1 (Veritas, Ltd.), or TeSR2 (Veritas, Ltd.) may be preferably used. In addition, it is preferable to use culture media used for culturing human iPS cells and/or human ES cells.

The culture medium used in the present invention preferably further comprises at least one growth factor. The liquid culture medium preferably contains at least one growth factor, which is not limited to the following, selected from the group consisting of FGF2 (basic fibroblast growth factor-2), TGF-β1 (transforming growth factor-β1), Activin A, IGF-1, MCP-1, IL-6, PAI, PEDF, IGFBP-2, LIF (Leukemia Inhibitory Factor), and IGFBP-7. LIF has a differentiation suppression effect, thus it is preferable that LIF is added to the culture medium in a predetermined amount. It is also preferable that the growth factor is FGF2 and/or TGF-β1.

The most preferable culture medium used in the present invention is a serum-free medium comprising, in addition to the cell cycle arresting agent described below, L-ascorbic acid, insulin, transferrin, selenium, and sodium bicarbonate as well as at least one growth factor. Particularly preferred is a serum-free DMEM/F12 medium comprising L-ascorbic acid, insulin, transferrin, selenium, and sodium bicarbonate as well as at least one growth factor (e.g., LIF, FGF2 and TGF-β1). Examples of such a medium that can be preferably used include, but not limited to, Essential 8™ medium (Life Technologies Japan Ltd.) supplemented with a ROCK inhibitor (see <5. Rho-kinase Inhibitors>). The Essential 8™ medium may be prepared by mixing DMEM/F-12 (HAM) (1:1), which is a DMEM/F12 medium marketed by Life Technologies Japan Ltd., and Essential 8™ supplement (containing L-ascorbic acid, insulin, transferrin, selenium, sodium bicarbonate, FGF2, and TGF-β1). Since the ROCK inhibitor has the effect of suppressing cell death of pluripotent stem cells, it is preferable that the ROCK inhibitor is added to the culture medium in a predetermined amount.

The culture medium used in the present invention may also comprise further components such as fatty acids or lipids, amino acids (e.g., non-essential amino acids), vitamins, cytokines, antioxidants, 2-mercaptoethanol, pyruvic acid, buffers, inorganic salts, and antibiotics. Examples of the antibiotics that can be used include penicillin, streptomycin, and amphotericin B.

### <4. Cell cycle arresting agents>

It is characteristic in the present invention that, when the pluripotent stem cells are subjected to suspension culture, at least one cell cycle arresting agent, e.g. a substance or agent that arrests the cell cycle by regulating cytoskeletons, is added to the culture medium to suppress cell aggregation of the stem cells. If the size of cell aggregates becomes too large due to cell aggregation, oxygen, nutritional components and the like are not supplied sufficiently to inside of the aggregates, and as a result, cell proliferation is significantly suppressed. For this reason, it is important to regulate the culture conditions such that the aggregates have a size that shows substantially no or little inhibition against the cell proliferation. In the present invention, the addition of a predetermined amount of a cell cycle arresting agent to the culture medium is effective to suppress such cell aggregation.

The cell cycle, as generally known, consists of four phases: G₁ phase which is in preparation for DNA replication; S phase which is in DNA replication; G₂ phase which is in preparation for cell division; and M phase which is in cell division. According to the present invention, a substance or agent capable of arresting the cell cycle may be used to suppress aggregation of pluripotent stem cells.

The cell cycle arresting agent includes any cytoskeletal regulating agent capable of suppressing aggregation of pluripotent stem cells in suspension culture, in other word, which is a substance or agent that arrests the cell cycle by regulating cytoskeletons,. Non-limiting examples of such cell cycle arresting agents include actin polymerization inhibitors, actin fiber-depolymerization inhibitors, tubulin polymerization inhibitors, and tubulin fiber-depolymerization inhibitors. Various substances (or agents) are known as these arresting agents. The cell cycle arresting phases (G₁ phase, G₂ phase, G₁/S phase, G₂/M phase, M phase, etc.) on which the arresting agents act are dependent on the substances (or agents). Thus, according to one embodiment of the present invention, the cell cycle arresting agent is at least one (i.e., one or more) substance(s) or agent(s) selected from G₁ phase-arresting agents, G₁/S phase-arresting agents, G₂ phase-arresting agents, G₂/M phase-arresting agents, and M phase-arresting agents.

Non-limiting examples of the cell cycle arresting agents that may be used in the present invention are listed as follows: colchicine, dimethylsulfoxide (DMSO), cytochalasin D, cytochalasin B, docetaxel, podophyllotoxin, demecolcine, nocodazole, jasplakinolide, vinblastine, griseofulvin, vinorelbine, latorunculin A, 2,3,5-triiodobenzoic acid (TIBA), ansamitocin P-3, cytochalasin A, cytochalasin E, indanocine, latorunculin B, wiskostatin, calpeptin (calpain inhibitor; G1/S), calpain inhibitor I; G1/S), acyclovir, aphidicolin, camptothecin, 5,6-dichloro-1-β-D-ribofuranosylbenzimidazole, emetine, etoposide, 5-fluorouracil, indole-3-carbinol, KN-93, L-mimosine, okadaic acid, vincristine, cyclin-dependent kinase inhibitors (cdk inhibitors) (e.g., kenpaullone), NSC625987, fascaplysin, ethyl-(6-hydroxy-4-phenylbenzo[4,5]furo[2,3-b])pyridine-3-carboxylate)), c-Myc inhibitors (e.g., 10058-F4; (Z,E)-5-(4-ethylbenzylidine)-2-thioxothiazolidin-4-one)), cyclophosphamide monohydrate, MDM2 antagonists (e.g., Nutlin-3), NSC66811, 2-benzyl-3-(4-chlorophenyl)-3-(3-hydroxypropoxy)-2,3-dihydroisoindol-1-one), p21-activated kinase inhibitors (e.g., PAK inhibitor, PAK18 (Zhao, L. et al., Nat. Neurosci. 2006; 9: 234-242; H₂N-RKKRRQRRR-G-PPVIAPRPEHTKSVYTRS-CO₂H (SEQ ID NO: 1)), Ras/Rac transformation blockers (e.g., SCH51344; 6-methoxy-4-(2-((2-hydroxyethoxyl)-ethyl)amino)-3-methyl-1H-pyrazolo[3,4-b]quinoline)), sodium 4-phenylbutyrate, STAT3 inhibitors (e.g., Ac-PpYLKTK-OH (SEQ ID NO: 2), PpYLKTK-mts (SEQ ID NO: 3, Turkson, J. et al., J. Biol. Chem. 2001; 276: 45443-45455), P3-AHNP-STAT3BP (H-YGRKKRRQR-G-FCDGFYACYKDV-PpYL-OH, cyclic (SEQ ID NO: 4), Tan, M. et al. Cancer Research 2006: 66(7): 3764-3771), 6-nitrobenzo[b]thiophene-1,1-dioxide; STAT3 inhibitory compounds, cyclin D1 inhibitors, or salts thereof or derivatives thereof.

As used herein, the "salt" is a salt that exhibits an effect of aggregation suppression on pluripotent stem cells and is formed from an acid or base, including an inorganic acid or base, or an organic acid or base. When the substance or compound is basic, the salt may be formed from an inorganic acid or an organic acid, or when the substance or compound is acidic, the salt may be formed from an inorganic base or an organic base. Examples of the inorganic and organic acids include, but are not limited to, inorganic acids such as hydrochloric acid, phosphoric acid, sulfuric acid, boric acid, and carbonic acid, and organic acids such as acetic acid, succinic acid, aspartic acid, glutamic acid, methanesulfonic acid, tartaric acid, and maleic acid. Examples of the inorganic and organic bases include, but are not limited to, alkali metals such as sodium and potassium, amines such as diethanolamine and ethylenediamine, and amino acids such as lysine and arginine.

Among the cell cycle arresting agents, non-limiting examples of the G₁ phase-arresting agent, G₁/S phase-arresting agent, G₂ phase-arresting agent, G₂/M phase-arresting agent, and M phase-arresting agent are shown below.

Examples of the G₁ phase-arresting agent include colchicine or dimethylsulfoxide (DMSO).

Examples of the G₁/S phase-arresting agent include cytochalasin D.

Examples of the G₂ phase-arresting agent include cytochalasin D, colchicine and docetaxel.

Examples of the G₂/M phase-arresting agent include podophyllotoxin, demecolcine, latorunculin A, cytochalasin B and nocodazole.

Examples of the M phase-arresting agent include jasplakinolide.

### <5. Rho-kinase Inhibitors>

It is preferable to add a substance that inhibits kinase activity of Rho-kinase (ROCK, Rho-associated protein kinase) and is referred to as a "ROCK inhibitor", to the culture medium in suspension culture of pluripotent stem cells for the purpose of suppressing cell death of the cells.

Examples of the ROCK inhibitor include: Y-27632 (4-[(1R)-1-aminoethyl]-N-pyridine-4-ylcyclohexane-1-carboxamide) or a salt thereof (e.g., dihydrochloride) (see, e.g., Ishizaki et al., Mol. Pharmacol. 57: 976-983 (2000); Narumiya et al., Methods Enzymol. 325: 273-284 (2000)), H-1152 ((S)-(+)-2-methyl-1-[(4-methyl-5-isoquinolinyl)sulfonyl]-hexahydro-1H-1,4-diazepine or a salt thereof (e.g., dihydrochloride)) (see, e.g., Sasaki et al., Pharmacol. Ther. 93: 225-232 (2002)), Fasudil/HA1077 (1-(5-isoquinolinsulfonyl)homopiperazine or a salt thereof (e.g., dihydrochloride)) (see, e.g., Uenata et al., Nature 389: 990-994 (1997)), Wf-536 ((+)-(R)-4-(1-aminoethyl)-N-(4-pyridyl)benzamide monohydrochloride) (see, e.g., Nakajima et al., CancerChemother. Pharmacol. 52(4): 319-324 (2003)) and derivatives thereof; and antisense nucleic acids against ROCK, RNA interference-inducing nucleic acid (e.g., siRNA), dominant negative mutants, and expression vectors thereof. In addition, other low-molecular-weight compounds have been known as the ROCK inhibitors, and such compounds or derivatives thereof may be used as the ROCK inhibitor in the present invention (see, e.g., published US Patent Application Nos. 2005/0209261 A1, 2005/0192304 A1, 2004/0014755 A1, 2004/0002508 A1, 2004/0002507 A1, 2003/0125344 A1, and 2003/0087919 A1, and WO 2003/062227 A1, WO 2003/059913 A1, WO 2003/062225 A1, WO 2002/076976 a1, and WO 2004/039796) A1. As the ROCK inhibitor, one or two or more ROCK inhibitors may be used.

The ROCK inhibitor is particularly preferably one or more inhibitors selected from Y-27632 and H-1152, and most preferably Y-27632. Y-27632 and H-1152 each may be used in the form of dihydrochloride.

The structural formula of Y-27632, i.e., 4-[(1R)-1-aminoethyl]-N-pyridin-4-ylcyclohexane-1-carboxamide described above is as follows:

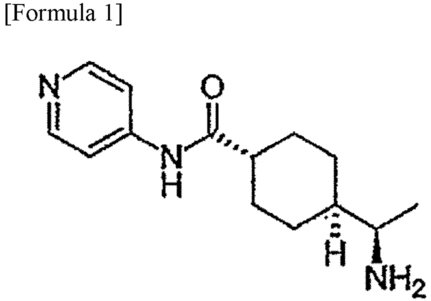

The structural formula of H-1152, i.e., (S)-(+)-2-methyl-1-[(4-methyl-5-isoquinolinyl)sulfonyl]-hexahydro-1H-1,4-diazepine described above is as follows:

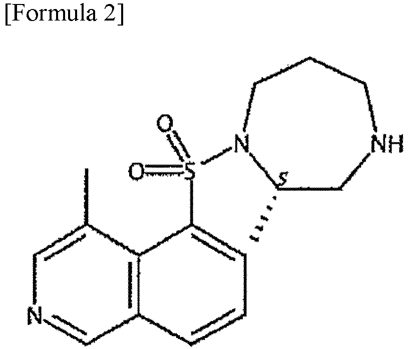

### <6. Methods for Suppressing Aggregation of Pluripotent Stem Cells>

Another aspect of the present invention is a method for suppressing aggregation of pluripotent stem cells, comprising a step of subjecting the pluripotent stem cells to suspension culture in a culture medium comprising a cell cycle arresting agent.

Hereinafter, specific embodiments of the step of culturing cells in suspension in a culture medium comprising a cell cycle arresting agent are described. The following procedures have to be performed using a sterile environment, sterile apparatuses and appliances, and the like to prevent contamination.

A predetermined number of pluripotent stem cells (e.g. 10⁴ to 10⁶ cells) are needed to be prepared before carrying out suspension culture. For this, the pluripotent stem cells to be used in suspension culture may be cultured to obtain a predetermined number of the cells in advance by conventional methods by subjecting the cells to maintenance culture while maintaining the undifferentiated state of the cells. The step of the maintenance culture herein is a step of culturing a cell population(s) before the suspension culture step or a cell aggregate(s) obtained after the suspension culture step or after a subsequent collection step, thereby to proliferate the cells while maintaining the undifferentiated state.

The usable culture as the maintenance culture is adhesion culture in which cells are cultured while adhered to a culture substrate, such as the surface of a culture vessel or a polymer support. The cells cultured by maintenance culture are detached from the culture substrate or detached from each other between cells, using, for example, known detaching agents (e.g., trypsin, collagenase, papain, Acutase™), and are then sufficiently dispersed and subjected to suspension culture. In order to disperse the cells, the cells may be passed through a strainer to single-cellulize them.

The predetermined number of pluripotent stem cells collected by the maintenance culture are subjected to the suspension culture step of the present invention as described below, and then to the collection step of the cells, where needed.

Specific embodiments of the cell cycle arresting agent, culture medium, and cells are as described above.

The concentration of the cell cycle arresting agent in the culture medium in the above-described steps may be appropriately adjusted depending on various conditions such as types of cells, the number of cells, and types of culture media, so that the aggregation of cells can be suppressed. The concentration of the cell cycle arresting agent in the culture medium in the above steps depends on types of the inhibitor, and the ranges of the concentration, in which the aggregation of pluripotent stem cells is suppressed in suspension culture, may be appropriately selected. This concentration is not limited, and the lower limit may be, for example, 3 ng/mL or more, 5 ng/mL or more, 7 ng/mL or more, 10 ng/mL or more, 20 ng/mL or more, 30 ng/mL or more, 40 ng/mL or more, 50 ng/mL or more, 100 ng/mL or more, 200 ng/mL or more, 300 ng/mL or more, 500 ng/mL or more, 1 µg/mL or more, 5 µg/mL or more, 10 µg/mL or more, 15 µg/mL or more, 20 µg/mL or more, 50 µg/mL or more, 70 µg/mL or more, or 100 µg/mL or more, and the upper limit may be, for example, 50 mg/mL or less, 40 mg/mL or less, 35 mg/mL or less, 30 mg/mL or less, 25 mg/mL or less, 20 mg/mL or less, 15 mg/mL or less, or 10 mg/mL or less. For example, the concentration may be in a range of 3 ng/mL or more and 30 mg/mL or less. Alternatively, the lower limit may be, for example, 3 nM or more, 5 nM or more, 10 nM or more, 15 nM or more, 20 nM or more, 25 nM or more, 30 nM or more, 40 nM or more, 50 nM or more, 80 nM or more, 100 nM or more, 300 nM or more, 500 nM or more, 1 µM or more, 2 µM or more, 3 µM or more, 4 µM or more, 5 µM or more, 10 µM or more, 20 µM or more, 30 µM or more, 50 µM or more, 60 µM or more, 70 µM or more, 80 µM or more, or 100 µM or more, and the upper limit may be, for example, 800 mM or less, 600 mM or less, 400 mM or less, 200 mM or less, 150 mM or less, 100 mM or less, 70 mM or less, 50 mM or less, 40 mM or less. For example, the concentration may be, 3 nM or more and 300 mM or less.

It is preferable that the ROCK inhibitor is added to the culture medium in the above steps to suppress cell death of pluripotent stem cells. The concentration of the ROCK inhibitor in the culture medium is desirably a level that does not affect the suppression of the aggregation of pluripotent stem cells. The lower limit includes, but is not limited to, for example, 3.3 ng/mL or more, 33 ng/mL or more, 330 ng/mL or more, 800 ng/mL or more, 1 µg/mL or more, 2 µg/mL or more, 3 µg/mL or more, 4 µg/mL or more, 5 µg/mL or more, 6 µg/mL or more, 7 µg/mL or more, 8 µg/mL or more, 9 µg/mL or more, or 10 µg/mL or more. The upper limit is not limited as long as the cells are not killed at the concentration, and includes, for example, 3.4 mg/mL or less, 340 µg/mL or less, 34 µg/mL or less, or 14 µg/mL or less. Alternatively, the lower limit of the concentration includes, but is not limited to, for example, 10 nM or more, 100 nM or more, 1 µM or more, 2.5 µM or more, 3 µM or more, 4 µM or more, 5 µM or more, 6 µM or more, 7 µM or more, 8 µM or more, 9 µM or more, 10 µM or more, 11 µM or more, or 12 µM or more, and the upper limit includes, but is not limited to, for example, 10 mM or less, 1 mM or less, 100 µM or less, 50 µM or less, or 40 µM or less. Alternatively, when the ROCK inhibitor is any of Y-27632 dihydrochloride, H-1152 dihydrochloride, HA10777 dihydrochloride, and Wf-536 monohydrochloride, the lower limit may be in a range of, for example, 270 ng/mL or more and 400 ng/mL or less, 1.3 µg/mL or more and 2.0 µg/mL or less, or 2.2 µg/mL or more and 3.2 µg/mL or less, and the upper limit may be in a range of, for example, 2.8 µg/mL or more and 4.0 µg/mL or less, 3.4 µg/mL or more and 4.7 µg/mL or less, 4.2 µg/mL or more and 5.9 µg/mL or less, or 14 µg/mL or more and 20 µg/mL or less.

As a criterion for determining that the aggregation of pluripotent stem cells has been suppressed in the present invention, the extent of cell aggregation (e.g., by observation with a microscope) in suspension culture in a culture medium comprising the same components except for being free of the cell cycle arresting agent and comprising ROCK inhibitors can be used as a comparative control.

The culture vessel used in the steps described above is preferably a vessel on which cells adhere less to the inner surface thereof. In order to obtain such a vessel with low cell adhesion, the inner surface of the vessel may be subjected to a hydrophilic surface treatment with a biocompatible substance, a polymeric substance, or the like. Examples of the culture vessel used include, but are not particularly limited to, Nunclon™ Sphera (Thermo Fisher Scientific Inc.).

The shape of the culture vessel is not particularly limited, and examples thereof include wells, dishes, flasks (e.g., spinner flasks), bottles (e.g., roller bottles), multi-layered (e.g., cell factories) and other shaped culture vessels, radial flow reactors, hollow fiber reactors (where examples of their materials: polyethylene coated with ethylene-vinyl alcohol copolymer, and cellulose acetate), plastic-bag reactors (e.g., CultiLife™ Spin Bag using ethylene vinyl acetate as the material (TAKARA BIO INC.), Vuelife FEP Bag 32-C using TFE-HFP copolymer (FEP) as the material (American Fluoroseal Corporation), culture bag A-1000NL (Nipro Corporation) and other bioreactors, and preferably bioreactors suitable for large scale culture. It is preferable that any of the bioreactors or vessels is in a type that reduces or suppresses shear stress on pluripotent stem cells as much as possible and reduces cell death as much as possible.

The suspension culture may be static culture or may be any culture performed under conditions in which the culture medium flows, but preferably the culture performed under conditions in which the culture medium flows. When the culture is performed under conditions in which a culture medium flows, the culture is preferably performed under the conditions in which the culture medium flows so as to suppress cell aggregation. Examples of the culture performed under conditions in which the culture medium flows so as to suppress aggregation of cells, include the culture performed under conditions in which the culture medium flows such that cells are concentrated on a spot due to stress (e.g., centrifugal force or centripetal force) caused by a flow such as a swirling flow or rocking flow; and the culture performed under conditions in which the culture medium flows due to linear back and forth movement, and the particularly preferred is the culture performed using swirling flow and/or rocking flow.

The swirling culture may be carried out by swirling a culture vessel containing a cell culture composition that comprises a culture medium, cells, and a cell cycle arresting agent, in a manner to draw a closed orbit such as a circle, ellipse, flattened circle, flattened ellipse or figure eight, along a generally horizontal plane, or by combining swirling and rocking or swirling and shaking. The speed of swirling is not particular limited, and the upper limit may be preferably 200 rpm or less, for example, 150 rpm or less, 120 rpm or less, 115 rpm or less, 110 rpm or less, 105 rpm or less, 100 rpm or less, 95 rpm or less, or 90 rpm or less. The lower limit may be preferably 1 rpm or more, for example, 10 rpm or more, 50 rpm or more, 60 rpm or more, 70 rpm or more, 80 rpm or more, or 90 rpm or more. The swirling width during the swirling culture is not particularly limited, and the lower limit may be, for example, 1 mm or more, 10 mm or more, 20 mm or more, or 25 mm or more. The swirling width during the swirling culture is not particularly limited, and the upper limit of the swirling width may be, for example, 200 mm or less, 100 mm or less, 50 mm or less, 30 mm or less, or 25 mm or less. Furthermore, the radius of rotation during the swirling culture is not particularly limited, and preferably set such that the swirling width is within the above-described range. The lower limit of the radius of rotation may be, for example, 5 mm or more, or 10 mm or more, and the upper limit thereof may be, for example, 100 mm or less, or 50 mm or less. Setting the swirling culture condition to these ranges can achieve producing cell aggregates with an appropriate size.

The rocking culture is the culture performed in the manner of flowing a liquid culture medium under rocking agitation, and it is carried out such that a culture vessel containing a cell culture composition that comprises a culture medium, cells, and a cell cycle arresting agent is rocked in a plane vertical to a generally horizontal plane. The speed of rocking is not particularly limited, and for example, when one round trip is set as one time, the rocking may be carried out with the lower limit of 2 times or more, 4 times or more, 6 times or more, 8 times or more, or 10 times or more per minute, and with the upper limit of 15 times or less, 20 times or less, 25 times or less, or 50 times or less per minute. During rocking, it is preferable to impart some angle relative to the vertical surface, i.e., a guiding angle, to the culture vessel. The rocking angle is not particularly limited, and, for example, the lower limit may be 0° or more, 1° or more, 2° or more, 4° or more, 6° or more, or 8° or more, and the upper limit may be 10° or less, 12° or less, 15° or less, 18° or less, or 20° or less. Setting the rocking culture condition to these ranges is preferable because cell aggregates with an appropriate size can be produced.

Further, the culture may be performed using mixing by the movement in which the above-mentioned rotary shaking and rocking are combined.

Culture using spinner flask-shaped culture vessel in which mixing blades are placed may be carried out. During this culture, the liquid culture medium is mixed by the mixing blades. The speed of rotation and the amount of culture medium are not particularly limited. When a commercially available spinner flask-shaped culture vessel is used, the amount recommended by the manufacturer may be suitably used as the amount of cell culture composition. The speed of rotation includes, but is not limited to, for example, 10 rpm or more and 100 rpm or less.

Preferably, the bioreactor comprises a vessel that is in the shape of a tank, column, back or the like and is equipped with an agitation system such as shaking, rocking or rotation, and further comprises a culture medium supply and elimination system, an oxygen/carbon dioxide supplying system, a monitoring system including sensors to measure a temperature, culture medium components, gas components or the like, a cell collection system, and the like. Furthermore, it is preferable that the bioreactor is fully automated. Any of batch type, semi-continuous type or continuous type may be employed as the culture type.

The seeding density of cells in a culture medium in suspension culture (i.e., the cell density at the start of suspension culture) may be adjusted appropriately and is not limited to the following. The lower limit of the seeding density is, for example, 0.01 × 10⁵ cells/mL or more, 0.1 × 10⁵ cells/mL or more, or 1 × 10⁵ cells/mL or more. The upper limit of the seeding density is, for example, 20 × 10⁵ cells/mL or less and 10 × 10⁵ cells/mL or less. When the seeding density is within the above-mentioned ranges, cell aggregates with an appropriate size are likely to be formed. For example, the seeding density may be 0.1 × 10⁵ cells/mL, 0.2 × 10⁵ cells/mL, 0.3 × 10⁵ cells/mL, 0.4 × 10⁵ cells/mL, 0.5 × 10⁵ cells/mL, 0.6 × 10⁵ cells/mL, 0.7 × 10⁵ cells/mL, 0.8 × 10⁵ cells/mL, 0.9 × 10⁵ cells/mL, 1 × 10⁵ cells/mL, 1.5 × 10⁵ cells/mL, 2 × 10⁵ cells/mL, 3 × 10⁵ cells/mL, 4 × 10⁵ cells/mL, 5 × 10⁵ cells/mL, 6 × 10⁵ cells/mL, 7 × 10⁵ cells/mL, 8 × 10⁵ cells/mL, 9 × 10⁵ cells/mL, or 10 × 10⁶ cells/mL.

The amount of the cell culture composition during suspension culture may be appropriately adjusted depending on the inner volume of the culture vessel or bioreactor used, but is, without limiting, for example, 1 mL or more and 100 L or less. Alternatively, when the culture vessel or bioreactor is a well plate, Erlenmeyer flask or culture bag, examples thereof include, without limiting, the following. For example, when a 12-well plate (with a bottom area per well of 3.5 cm² in a flat view) is used, the volume may be, for example, 0.5 ml/well or more and 1.5 ml/well or less, and may be, for example, 1.3 ml/well. For example, when a 6-well plate (with a bottom area per well of 9.6 cm² in a flat view) is used, the volume may be, for example, 1.5 mL/well or more, 2 mL/well or more, or 3 mL/well or more, and may be, for example, 6.0 mL/well or less, 5 mL/well or less, or 4 mL/well or less. For example, when a 125-mL Erlenmeyer flask (an Erlenmeyer flask with a volume of 125 mL) is used, the volume may be, for example, 10 mL/flask or more, 15 mL/flask or more, 20 mL/flask or more, 25 mL/flask or more, 20 mL/flask or more, 25 mL/flask or more, or 30 mL/flask or more, and may be, for example, 50 mL/flask or less, 45 mL/flask or less, or 40 mL/flask or less. For example, when a 500-mL Erlenmeyer flask (an Erlenmeyer flask with a volume of 500 mL) is used, the volume may be, for example, 100 mL/flask or more, 105 mL/flask or more, 110 mL/flask or more, 115 mL/flask or more, 120 mL/flask or more, and may be, for example, 150 mL/flask or less, 145 mL/flask or less, 140 mL/flask or less, 135 mL/flask or less, 130 mL/flask or less, or 125 mL/flask or less. For example, when a 1000-mL Erlenmeyer flask (an Erlenmeyer flask with a volume of 1000 mL) is used, the volume may be, for example, 250 mL/flask or more, 260 mL/flask or more, 270 mL/flask or more, 280 mL/flask or more, or 290 mL/flask or more, and may be, for example, 350 mL/flask or less, 340 mL/flask or less, 330 mL/flask or less, 320 mL/flask or less, or 310 mL/flask or less. For example, when a 2000-mL Erlenmeyer flask (an Erlenmeyer flask with a volume of 2000 mL) is used, the volume may be, for example, 500 mL/flask or more, 550 mL/flask or more, or 600 mL/flask or more, and may be, for example, 1000 mL/flask or less, 900 mL/flask or less, 800 mL/flask or less, or 700 mL/flask or less. For example, when a 3000-mL Erlenmeyer flask (an Erlenmeyer flask with a volume of 3000 mL) is used, the volume may be, for example, 1000 mL/flask or more, 1100 mL/flask or more, 1200 mL/flask or more, 1300 mL/flask or more, 1400 mL/flask or more, or 1500 mL/flask or more, and may be, for example, 2000 mL/flask or less, 1900 mL/flask or less, 1800 mL/flask or less, 1700 mL/flask or less, or 1600 mL/flask or less. For example, when a 2-L culture bag (a disposable culture bag with a volume of 2 L) is used, the volume may be, for example, 100 mL/bag or more, 200 mL/bag or more, 300 mL/bag or more, 400 mL/bag or more, 500 mL/bag or more, 600 mL/bag or more, 700 mL/bag or more, 800 mL/bag or more, 900 mL/bag or more, or 1000 mL/bag or more, and may be, for example, 2000 mL/bag or less, 1900 mL/bag or less, 1800 mL/bag or less, 1700 mL/bag or less, 1600 mL/bag or less, 1500 mL/bag or less, 1400 mL/bag or less, 1300 mL/bag or less, 1200 mL/bag or less, or 1100 mL/bag or less. For example, when a 10-L culture bag (a disposable culture bag with a volume of 10 L) is used, the volume may be, for example, 500 mL/bag or more, 1 L/bag or more, 2 L/bag or more, 3 L/bag or more, 4 L/bag or more, or 5 L/bag or more, and may be, for example, 10 L/bag or less, 9 L/bag or less, 8 L/bag or less, 7 L/bag or less, or 6 L/bag or less. For example, when a 20-L culture bag (a disposable culture bag with a volume of 20 L) is used, the volume may be, for example, 1 L/bag or more, 2 L/bag or more, 3 L/bag or more, 4 L/bag or more, 5 L/bag or more, 6 L/bag or more, 7 L/bag or more, 8 L/bag or more, 9 L/bag or more, or 10 L/bag or more, and may be, for example, 20 L/bag or less, 19 L/bag or less, 18 L/bag or less, 17 L/bag or less, 16 L/bag or less, 15 L/bag or less, 14 L/bag or less, 13 L/bag or less, 12 L/bag or less, or 11 L/bag or less. For example, when a 50-L culture bag (a disposable culture bag with a volume of 50 L) is used, the volume may be, for example, 1 L/bag or more, 2 L/bag or more, 5 L/bag or more, 10 L/bag or more, 15 L/bag or more, 20 L/bag or more, or 25 L/bag or more, and may be, for example, 50 L/bag or less, 45 L/bag or less, 40 L/bag or less, 35 L/bag or less, or 30 L/bag or less. When the amount of the cell culture composition is within these ranges, cell aggregates with an appropriate size are likely to be formed. Alternatively, when another larger volume of bioreactor is used, the amount of the cell culture composition may be greater than the upper limit of the amount of the cell culture composition as exemplified above, the amount of which may vary depending on the volume of the bioreactor.

The volume of culture vessel used may be appropriately selected and includes, but is not particularly limited, for example, 2 mL or more and 200 L or less. Alternatively, when the area of the bottom of a portion containing a liquid culture medium is determined in a flat view, the culture vessel in which a lower limit of the area is, for example, 0.32 cm² or more, 0.65 cm² or more, 0.65 cm² or more, 1.9 cm² or more, 3.0 cm² or more, 3.5 cm² or more, 9.0 cm² or more, or 9.6 cm² or more may be used, and the culture vessel in which the upper limit of the area is, for example, 1000 cm² or less, 500 cm² or less, 300 cm² or less, 150 cm² or less, 75 cm² or less, 55 cm² or less, 25 cm² or less, 21 cm² or less, 9.6 cm² or less, or 3.5 cm² or less may be used. Alternatively, when another bioreactor with larger volume is used, the area may be greater than the upper limit of the area of the bottom as exemplified above, the area of which may vary depending on the shape of the bioreactor.

Conditions such as the temperature, culture period, CO₂ level in suspension culture of the cells in the presence of the lipid are not particularly limited. The culture temperature is not limited, and the lower limit is, for example, 20°C or more, 25°C or more, 30°C or more, 35°C or more, and the upper limit is, for example, 45°C or less, or 40°C or less, and preferably the temperature is 37°C. The culture period is not limited, and the lower limit is, for example, 0.5 hours or more, 12 hours or more, 1 day or more, or 2 days or more, and the upper limit is, for example, 7 days or less, 6 days or less, 5 days or less, 4 days or less, or 3 days or less. The CO₂ level during culture is not limited, and the lower limit is, for example, 4% or more, 4.3% or more, or 4.5% or more, and the upper limit is, for example, 10% or less, 8% or less, 7% or less, or 6% or less, and preferably the CO₂ level is 5%. The suspension culture may include passage procedures. When the culture conditions are within these ranges, cell aggregates with an appropriate size are likely to be formed. When the culture conditions are within these ranges, cell aggregates with an appropriate size are likely to be formed.

Furthermore, the culture medium can be exchanged in an appropriate frequency. The frequency of the culture medium exchange may vary depending on the kind of cells to be cultured, but, for example, may be one or more times every 5 days, one or more times every 4 days, one or more times every 3 days, one or more times every 2 days, one or more times a day. The preferred frequency of the culture medium exchange is, for example, one or more times a day. For the culture medium exchange, after collecting cells in the same manner as in the collection step described below, a fresh culture medium is added, cell aggregates are gently dispersed, and then culturing is performed again. In the suspension culture step, to what extent the number of cells is increased and to what the state of cells is adjusted may be determined appropriately depending on the kind of cells to be cultured, the purpose of cell aggregation, the kind of culture medium, and the culture conditions. Alternatively, the culture medium may be exchanged continuously or periodically when a culture apparatus in which the culture medium flows is used. This frequency of the culture medium exchange is particularly suitable when cell aggregates of pluripotent stem cells are cultured. Methods of the culture medium exchange include, but are not particularly limited, for example, a method of separating cells from the culture medium via a porous membrane and replenishing a fresh medium, and, in the case where the culture medium flows, a method of draining old medium from the circulatory system and replenishing a fresh medium continuously or at fixed time intervals. There is also a method of separating cells from the culture medium by centrifugation, but this method requires suppressing cell death.

It is preferable that the cells used in the suspension culture step are cells that are cultured by a maintenance culture step in advance, collected by a collection step, and then single-cellularized, where needed. The maintenance culture step, the collection step, and the single-cellularization will be described below.

After the suspension culture step, the culture solution is discarded by conventional procedures, and the cells are collected. At this time, the cells are preferably collected as single-cellularized cells by a detachment or dispersion treatment. Specific methods thereof will be described in detail in the collection step described below. The collected cells may be directly, or after being washed with a buffer (e.g., PBS buffer), saline, or culture medium (preferably, a culture medium used in the next step or a basal medium) where needed, subjected to the next step.

### (Maintenance Culture Step)

A "maintenance culture step" is a step of culturing a cell population before a suspension culture step, or a cell aggregate obtained after a suspension culture step or after a subsequent collection step in order to proliferate the cells while remaining undifferentiated. The maintenance culture may be the adhesion culture in which cells are cultured while adhered to a culture substrate such as a vessel or a support, or may be the suspension culture in which cells are subjected to suspension culture in a culture medium.

In the maintenance culture step, cells of interest may be cultured by animal cell culture methods known in the art. The culture performed in the maintenance culture step may be the adhesion culture or the suspension culture.

Specific embodiments of the culture medium and cells used in the maintenance culture step are as described above.

The culture vessel, seeding density of cells, and culture conditions used in the maintenance culture step are as described above with respect to the suspension culture step.

The flow state of the culture medium in the maintenance culture step is not limited. The maintenance culture may be either static culture or fluid culture.

The "static culture" refers to culturing cells while standing the culture medium in a culture vessel. In adhesion culture, this static culture is typically employed.

The "fluid culture" refers to culturing cells under conditions in which the culture medium is flowed. Specific embodiments of the fluid culture are as described above with respect to the suspension culture step.

In the maintenance culture step, to what extent the number of cells is increased and to what the state of cells is adjusted may be determined appropriately depending on the type of cells to be cultured, the purpose of cell aggregation, the type of culture medium, and the culture conditions.

A preferable aspect of the maintenance culture step is the step comprising further culturing cell aggregates formed by performing a suspension culture step in the presence of a suppressor of cell aggregation that comprises a cell cycle arresting agent described above. According to this aspect, the culture method of the maintenance culture step includes, but is not particularly limited, a step of subjecting cell aggregates to suspension culture in a culture medium free of the suppressor of cell aggregation comprising a cell cycle arresting agent. As the culture medium used for the maintenance culture, the same culture medium as described above but free of the suppressor of cell aggregation comprising a cell cycle arresting agent may be used. As the conditions for the maintenance culture, the same conditions as those for the suspension culture step may be used. According to this aspect for the maintenance culture step, preferably the culture medium is exchanged in an appropriate frequency. The frequency of the culture medium exchange may vary depending on the cell species, but a culture medium exchange procedure may be included at the frequency of, for example, one or more times every 5 days, one or more times every 4 days, one or more times every 3 days, one or more times every 2 days, one or more times a day. This frequency of the culture medium exchange is particularly suitable when cell aggregates of stem cells are cultured. Methods of the culture medium exchange include, but are not particularly limited, for example, a method that may comprise: collecting the whole amount of the cell aggregate-containing cell culture composition into a centrifuge tube; subjecting the tube to centrifugation or a standing state for about 5 minutes; removing the supernatant from precipitated cell aggregates; then adding a fresh culture medium; gently dispersing the cell aggregates, and then returning the cell aggregate-dispersed culture medium to a culture vessel such as a plate, so that the cell aggregates may be cultured continuously. The culture period of the maintenance culture step in this aspect is not particularly limited, and may be, for example, 3 days or more and 7 days or less. By further subjecting the cell aggregates to suspension culture in a culture medium free of the suppressor of cell aggregation comprising a cell cycle arresting agent described above, under the conditions described above, it is possible to obtain cell aggregates with an appropriate size.

After the maintenance culture step, the culture solution is discarded by conventional procedures, and the cells are collected. At this time, the cells are preferably collected as single-cellularized cells by a detachment or dispersion treatment. Specific methods thereof are described in detail in the collection step described below. The collected cells may be directly, or after being washed with a buffer (e.g., PBS buffer), saline, or culture medium (preferably, a culture medium used in the next step or a basal medium) where needed, subjected to the next step.

### (Collection Step)

The "collection step" is a step of collecting cultured cells from a culture solution after a maintenance culture step or a suspension culture step, and is a step of collecting a cell aggregate(s) of interest by the method of the present invention.

As used herein, the "collection of cells" or simply "collection" refers to separating cells from a culture solution to obtain the cells. The collection method of cells may follow conventional procedures used in cell culture methods in the art, and is not particularly limited. The cell culture methods may generally be classified into the suspension culture method and the adhesion culture method. Hereinafter, the collection method of cells after each culture method will be described.

### (Collection Method After Suspension Culture Method)

When cells are cultured by the suspension culture method, the cells are present in a suspension state in the culture solution. Thus, collection of the cells can be accomplished by removing the liquid components of the supernatant in the static state or by centrifugation. Furthermore, the use of filters, hollow fiber separation membranes or the like may be selected as the collection method of cells.

When the liquid components are removed in the static state, the vessel containing the culture solution may be left in the static state for about 5 minutes, and the supernatant may be removed while leaving the precipitated cells and cell aggregates. Centrifugation may also be performed at a rotation speed and processing time at which cells are not damaged by centrifugal force. For example, the lower limit of the rotation speed is not particularly limited as long as the cells can be precipitated, and may be, for example, 500 rpm or more, 800 rpm or more, or 1000 rpm or more. While, the upper limit is not limited as long as the cells are not or hardly damaged by centrifugal force at the rotation speed, and may be, for example, 1400 rpm or less, 1500 rpm or less, or 1600 rpm or less. The lower limit of the processing time is not particularly limited as long as the cells can be precipitated at the rotation speed, and may be, for example, 30 seconds or more, 1 minute or more, 3 minutes or more, or 5 minutes or more. The upper limit is not limited as long as the cells are not or hardly damaged by the rotation, and may be, for example, 6 minutes or less, 8 minutes or less, or 10 minutes or less. The collected cells can be washed where needed. Methods for washing are not limited. For example, the same method as the washing method after the maintenance culture step described above may be used. A buffer (e.g., PBS buffer), saline, or culture medium (e.g., a basal medium) may be used as a washing solution.

### (Collection Method After Adhesion Culture)

When cells are cultured by an adhesion culture method, many cultured cells are present while adhered to external matrixes such as culture vessels and culture supports. As such, to remove the culture solution from the culture vessel, the culture vessel may be gently tilted after the culture to remove liquid components. Since the cells adhered to the external matrix remain in the culture vessel, the cells and the culture solution can be readily separated.

The cell surface adhered to the external matrix may then be washed where needed. A buffer (e.g., PBS buffer), saline, or culture medium (e.g., a basal medium) may be used as a washing solution. However, the washing solution is not limited thereto. The washing solution after washing may be removed in the same manner as the culture solution. This washing step may be repeated multiple times.

The cell population adhered to the external matrix is then detached from the external matrix. The detachment method may be performed in a manner known in the art. Typically, scraping, detaching agents containing proteolytic enzymes as active ingredients, chelating agents such as EDTA, or mixtures of detaching agents and chelating agents, or the like are used.

Scraping is a method for stripping cells attached to an external matrix by mechanical means such as scrapers. However, since cells are easily damaged by mechanical procedures, when the collected cells are subjected to further culture, it is preferable to employ a detachment method which chemically destroys or degrades the scaffold portion of cells adhered tightly to an external matrix and releases the adhesion between the cells and the external matrix.

In the detachment method, a detaching agent and/or a chelating agent are used. The detaching agent includes, but is not limited to, for example, trypsin, collagenase, pronase, hyaluronidase, elastase, as well as commercially available Accutase (registered trade mark), TrypLE™ Express Enzyme (Life Technologies Japan Ltd.), TrypLE™ Select Enzyme (Life Technologies Japan Ltd.), "Dispase" (registered trade mark). The concentration and processing time of each detaching agent may be in the ranges of those in conventional procedures for cell detachment or dispersion. For example, when the detaching agent is trypsin, the lower limit of the concentration in the solution is not particularly limited as long as the cells can be detached at the concentration, and may be, for example, 0.01% or more, 0.02% or more, 0.03% or more, 0.04% or more, 0.05% or more, 0.08% or more, or 0.10% or more. The upper limit of the concentration in the solution is not particularly limited as long as cells themselves are not affected (e.g., lysed) by the action of trypsin at the concentration, and may be, for example, 0.15% or less, 0.20% or less, 0.25% or less, or 0.30% or less. The processing time also depends on the concentration of trypsin, but the lower limit is not particularly limited as long as the cells can be sufficiently detached from the external matrix by the action of trypsin at the time, and may be, for example, 1 minute or more, 2 minutes or more, 3 minutes or more, 4 minutes or more, or 5 minutes or more. The upper limit of the processing time is not particularly limited as long as cells themselves are not affected (e.g., lysed) by the action of trypsin at the time, and may be, for example, 8 minutes or less, 10 minutes or less, 12 minutes or less, 15 minutes or less, 18 minutes or less, or 20 minutes or less. Other detaching agents and chelating agents can also be used generally in the same manner as described above. When commercially available detaching agents are used, the concentrations and processing times described in the attached protocol can be employed.

The cells detached from the external matrix are separated from the supernatant containing detaching agents by centrifugation. The centrifugal conditions may be the same as those in the "Collection Method After Suspension Culture Method" described above. The collected cells may be washed where needed. The washing method may also be carried out in the same manner as those in the "Collection Method After Suspension Culture Method" described above.

The cells obtained after this step may partially include cell assemblies , such as monolayer cell pieces and cell aggregates. The collected cells may be treated to single-cellularize them, where needed.

### (Single-cellularization)

As used herein, "single-cellularization" refers to dispersing cell assemblies, such as monolayer cell pieces and cell aggregates, in which multiple cells are adhered or aggregated to each other and thereby to form the state of single free cells.

The single-cellularization may be performed by increasing the concentration of detaching agents and/or chelating agents, and/or, by extending the processing time with detaching agents and/or chelating agents used in the above-described detachment method. For example, when the detaching agent is trypsin, the lower limit of the concentration in the solution is not particularly limited as long as the cell assemblies can be dispersed at the concentration, and may be, for example, 0.15% or more, 0.18% or more, 0.20% or more, or 0.24% or more. The upper limit of the concentration in the solution is not particularly limited as long as the cells themselves are not affected (e.g., lysed) at the concentration, and may be, for example, 0.25% or less, 0.28% or less, or 0.30% or less. The processing time also depends on the concentration of trypsin, but the lower limit is not particularly limited, as long as the cell assemblies can be sufficiently dispersed by the action of trypsin at the time, and may be, for example, 5 minutes or more, 8 minutes or more, 10 minutes or more, 12 minutes or more, or 15 minutes or more. The upper limit of the processing time is not particularly limited as long as the cells themselves are not affected (e.g., lysed) by the action of trypsin at the time, and may be, for example, 18 minutes or less, 20 minutes or less, 22 minutes or less, 25 minutes or less, 28 minutes or less, or 30 minutes or less. When commercially available detaching agents are used, the agents may be used at the concentration at which the cells can be dispersed to be a single sate as described in the attached protocol. The single-cellularization may be facilitated by physically treating cells in a gentle way after treating with the detaching agent and/or chelating agent. This physical treatment includes, but is not limited to, for example, a method of pipetting cells together with the solution multiple times. Additionally, where needed, the cells may be passed through a strainer or mesh.

The single-cellularized cells can be collected by removing the supernatant containing a detaching agent by standing or centrifugation. The collected cells may be washed where needed. Conditions for centrifugation and methods for washing may be carried out in the same manner as in the "Collection Method After Suspension Culture Method" described above.

### <7. Suppressor of pluripotent stem cell aggregation>

Another aspect of the present invention is a suppressor of pluripotent stem cell aggregation for use in suspension culture of pluripotent stem cells, comprising a cell cycle arresting agent.

The suppressor of pluripotent stem cell aggregation of the present invention can be used to appropriately suppress aggregation of cells in a suspension culture system to form cell aggregates with a substantially uniform size. In the suspension culture of stem cells using the suppressor of pluripotent stem cell aggregation of the present invention, it is possible to maintain the undifferentiated state of the stem cells.

The form of the suppressor of pluripotent stem cell aggregation according to the present invention is not particularly limited, and may be the cell cycle arresting agent itself or may be a composition comprising the cell cycle arresting agent in combination with other components.

The kinds of other components are not particularly limited, and examples thereof include solvents, excipients, and/or compounds having other pharmacological effects or actions. These components may be natural products or may be non-natural substances. The composition resulted from the said combination may be a naturally occurring composition or may be an artificial composition. The artificial composition may be a composition of which at least one component is a non-natural substance. Examples of the non-natural substance include synthetic compounds. Specific examples of synthetic compounds that may be contained in the suppressor of cell aggregation include ROCK inhibitors (e.g., Y-27632) used for suppression of cell death in the examples described below.

The form of the composition includes, but is not particularly limited to, for example, the form of a culture medium used for suspension culture or the form of a composition added upon preparation of a culture medium.

According to a preferred embodiment of the present invention, the suppressor of pluripotent stem cell aggregation is a culture medium or a buffer like phosphate buffer, comprising a cell cycle arresting agent. Examples of the concentration of the cell cycle arresting agent in the culture medium include the concentrations of the cell cycle arresting agent in a culture medium described for suspension culture in the section <6. Methods for Suppressing Aggregation of Pluripotent Stem Cells>.

Another embodiment of the suppressor of pluripotent stem cell aggregation according to the present invention is a liquid or solid composition comprising a cell cycle arresting agent in a liquid or solid medium. The liquid or solid composition is an additive which is added when a culture medium for suspension culture is prepared. Preferably, the suppressor of pluripotent stem cell aggregation in this embodiment is prepared in an enriched (or concentrated) form such that the final concentration of the cell cycle arresting agent in the culture medium to be prepared is a concentration of the cell cycle arresting agent in the culture medium described for suspension culture in the section <6. Methods for Suppressing Aggregation of Pluripotent Stem Cells>. The concentration of the cell cycle arresting agent in the suppressor of pluripotent stem cell aggregation in this embodiment is not particularly limited, and is, for example, 1-fold or more, 2-fold or more, 10-fold or more, 100-fold or more, 1000-fold or more, or 10000-fold or more of, for example, the concentration of the cell cycle arresting agent described above as the preferred concentration in the culture medium during suspension culture. The concentration depends on the solubility to the solvent (e.g., DMSO, EtOH, or buffer) for solubilizing the suppressor of pluripotent stem cell aggregation, and non-limiting examples thereof include 55.0 mg/mL or more and 1.1 g/mL or less. The lower limit of the concentration of the cell cycle arresting agent in one embodiment is not particularly limited as long as the suppression effect on cell aggregation is obtained at the said concentration, and may be, for example, 60 mg/mL or more, 70 mg/mL or more, 80 mg/mL or more, 100 mg/mL or more, 110 mg/mL or more, 130 mg/mL or more, 140 mg/mL or more, 150 mg/mL or more, 160 mg/mL or more, 180 mg/mL or more, 200 mg/mL or more, 300 mg/mL or more, or 500 mg/mL or more, and the upper limit may be, for example, 600 mg/mL or less, 700 mg/mL or less, 800 mg/mL or less, 900 mg/mL or less, or 1 g/mL or less. Alternatively, the lower limit may be, for example, 60 mM or more, 70 mM or more, 80 mM or more, 100 mM or more, 110 mM or more, 130 mM or more, 140 mM or more, 150 mM or more, 160 mM or more, 180 mM or more, 200 mM or more, 300 mM or more, 500 mM or more, or 700 mM or more, and the upper limit is not particularly limited as long as the cells are not to be killed at the concentration, and may be, for example, 15 M or less, 10 M or less, 5 M or less, or 1 M or less. For example, the concentration may be in a range of 60 mM or more and 15 M or less.

In addition to the cell cycle arresting agent, the suppressor of pluripotent stem cell aggregation may also comprise, as additives, antibiotics, buffers, thickeners, colorants, stabilizers, surfactants, emulsifiers, preservatives, preserving agents, antioxidants, and the like. Examples of the antibiotics that can be used include, but are not particularly limited to, penicillin, streptomycin, and amphotericin B. Examples of the buffer include phosphate buffer, tris-hydrochloric acid buffer, and glycine buffer. Examples of the thickener include gelatin and polysaccharides. Examples of the colorant include Phenol Red. Examples of the stabilizer include albumin, dextran, methyl cellulose, and gelatin. Examples of the surfactant include cholesterol, an alkyl glycoside, alkyl polyglycoside, alkyl monoglyceride ether, glucoside, maltoside, neopentyl glycol series, polyoxyethylene glycol series, thioglucoside, thiomaltoside, peptide, saponin, phospholipid, sorbitan fatty acid ester, and fatty acid diethanolamide. Examples of the emulsifier include a glycerin fatty acid ester, sorbitan fatty acid ester, propylene glycol fatty acid ester, and sucrose fatty acid ester. Examples of the preservative include aminoethyl sulfonic acid, benzoic acid, sodium benzoate, ethanol, sodium edetate, agar, dl-camphor, citric acid, sodium citrate, salicylic acid, sodium salicylate, phenyl salicylate, dibutylhydroxy toluene, sorbic acid, potassium sorbate, nitrogen, dehydro acetic acid, sodium dehydroacetate, 2-naphthol, white soft sugar, honey, isobutyl paraoxybenzoate, isopropyl paraoxybenzoate, ethyl paraoxybenzoate, butyl paraoxybenzoate, propyl paraoxybenzoate, methyl paraoxybenzoate, 1-menthol, and eucalyptus oil. Examples of the preserving agent include benzoic acid, sodium benzoate, ethanol, sodium edetate, dried sodium sulfite, citric acid, glycerin, salicylic acid, sodium salicylate, dibutylhydroxy toluene, D-sorbitol, sorbic acid, potassium sorbate, sodium dehydroacetate, isobutyl paraoxybenzoate, isopropyl paraoxybenzoate, ethyl paraoxybenzoate, butyl paraoxybenzoate, propyl paraoxybenzoate, methyl paraoxybenzoate, propylene glycol, and phosphoric acid. Examples of the antioxidant include citric acid, citric acid derivatives, vitamin C and derivatives thereof, lycopene, vitamin A, carotenoids, vitamin B and derivatives thereof, flavonoids, polyphenols, glutathione, selenium, sodium thiosulfate, vitamin E and derivatives thereof, α-lipoic acid and derivatives thereof, pycnogenol, flavangenol, super oxide dismutase (SOD), glutathione peroxidase, glutathione-S-transferase, glutathione reductase, catalase, ascorbic acid peroxidase, and mixtures thereof.

The suppressor of pluripotent stem cell aggregation may comprise a growth factor, for example, may comprise one or more growth factors of FGF2 and TGF-β1.

### <8. Method for Producing Cell Aggregates, and Cell Aggregates Produced Thereby>

Another aspect of the present invention is a method for producing cell aggregates, comprising a step of subjecting pluripotent stem cells to suspension culture in a culture medium comprising a cell cycle arresting agent, for example, at the concentrations shown below, but not limited thereto.

Regarding the concentration of the cell cycle arresting agent in the culture medium, the lower limit may be, for example, 3 ng/mL or more, 5 ng/mL or more, 7 ng/mL or more, 10 ng/mL or more, 20 ng/mL or more, 30 ng/mL or more, 40 ng/mL or more, 50 ng/mL or more, 100 ng/mL or more, 200 ng/mL or more, 300 ng/mL or more, 500 ng/mL or more, 1 µg/mL or more, 5 µg/mL or more, 10 µg/mL or more, 15 µg/mL or more, 20 µg/mL or more, 50 µg/mL or more, 70 µg/mL or more, or 100 µg/mL or more, and the upper limit may be, for example, 50 mg/mL or less, 40 mg/mL or less, 35 mg/mL or less, 30 mg/mL or less, 25 mg/mL or less, 20 mg/mL or less, 15 mg/mL or less, or 10 mg/mL or less. For example, the concentration may be in the range of 3 ng/mL or more and 30 mg/mL or less. Alternatively, the lower limit may be, for example, 3 nM or more, 5 nM or more, 10 nM or more, 15 nM or more, 20 nM or more, 25 nM or more, 30 nM or more, 40 nM or more, 50 nM or more, 80 nM or more, 100 nM or more, 300 nM or more, 500 nM or more, 1 µM or more, 2 µM or more, 3 µM or more, 4 µM or more, 5 µM or more, 10 µM or more, 20 µM or more, 30 µM or more, 50 µM or more, 60 µM or more, 70 µM or more, 80 µM or more, or 100 µM or more, and the upper limit may be, for example, 800 mM or less, 600 mM or less, 400 mM or less, 200 mM or less, 150 mM or less, 100 mM or less, 70 mM or less, 50 mM or less, or 40 mM or less. For example, the concentration may be 3 nM or more and 300 mM or less.

According to the method of the present invention, cell aggregates with appropriate size can be produced in high yields while maintaining the undifferentiated state of the stem cells. Specifically, in the production experiments in the wells of the Examples described below, glucose consumption in the production methods of the present invention is about 1.3-fold or more on Day 5 of culture compared to that in culture in a control medium free of the cell cycle arresting agent (see Fig. 3), and the cell yield is also increased 5-fold or more than the seeded cell number (see Fig. 4).

Specific embodiments of the cell cycle arresting agent, culture medium and cell, and the concentration of the cell cycle arresting agent in the culture medium in the above step are as described above.

Specific embodiments of the step are similar to the specific embodiments of the "step of subjecting cells to suspension culture in a culture medium comprising a cell cycle arresting agent" in the method for suppressing aggregation of pluripotent stem cells described in the section <6. Methods for Suppressing Aggregation of Pluripotent Stem Cells>.

Yet another aspect of the present invention is a cell aggregate(s) obtained by the method for producing cell aggregate described above.

According to this aspect of the present invention, the cell aggregate has an appropriate size and a high cell viability (see Figs. 1A to 1C and Figs. 2B to 2E). Regarding the size range of cell aggregates of pluripotent stem cells that can be produced by the method of the present invention, when observed under a microscope, the range of the upper limit of the size of the widest portion in the observed images is, for example, 400 µm or more and 500 µm or less, 300 µm or more and 400 µm or less, or 200 µm or more and 300 µm or less. The range of the lower limit of the size of the narrowest portion is, for example, 50 µm or more and 70 µm or less, 70 µm or more and 100 µm or less, 100 µm or more and 150 µm or less, or 100 µm or more and less than 200 µm. The preferred size range of the cell aggregates of pluripotent stem cells that may be produced by the methods of the present invention is, for example, about 100 µm or more and about 300 µm or less. The cell aggregates with such a size range have a preferable cell growth environment because oxygen and nutritional components are easily supplied to inner cells of the aggregates. Furthermore, the cells constituting the cell aggregate remain undifferentiated.

According to this aspect of the present invention, preferably the cell aggregate has the features as described in the section <2. Cell Aggregates> in addition to the feature that the cell aggregate is produced by suspension culture in a culture medium comprising a cell cycle arresting agent.

### <9. Composition for Culture of Cells>

Another aspect of the present invention is a composition for culture of cells, comprising a cell aggregate(s) of pluripotent stem cells, a culture medium, and a cell cycle arresting agent, for example, at the concentrations shown below, but not limited thereto.

Regarding the concentration of the cell cycle arresting agent in the culture medium, the lower limit may be, for example, 3 ng/mL or more, 5 ng/mL or more, 7 ng/mL or more, 10 ng/mL or more, 20 ng/mL or more, 30 ng/mL or more, 40 ng/mL or more, 50 ng/mL or more, 100 ng/mL or more, 200 ng/mL or more, 300 ng/mL or more, 500 ng/mL or more, 1 µg/mL or more, 5 µg/mL or more, 10 µg/mL or more, 15 µg/mL or more, 20 µg/mL or more, 50 µg/mL or more, 70 µg/mL or more, or 100 µg/mL or more, and the upper limit may be, for example, 50 mg/mL or less, 40 mg/mL or less, 35 mg/mL or less, 30 mg/mL or less, 25 mg/mL or less, 20 mg/mL or less, 15 mg/mL or less, or 10 mg/mL or less. For example, the concentration may be in the range of 3 ng/mL or more and 30 mg/mL or less. Alternatively, the lower limit may be, for example, 3 nM or more, 5 nM or more, 10 nM or more, 15 nM or more, 20 nM or more, 25 nM or more, 30 nM or more, 40 nM or more, 50 nM or more, 80 nM or more, 100 nM or more, 300 nM or more, 500 nM or more, 1 µM or more, 2 µM or more, 3 µM or more, 4 µM or more, 5 µM or more, 10 µM or more, 20 µM or more, 30 µM or more, 50 µM or more, 60 µM or more, 70 µM or more, 80 µM or more, or 100 µM or more, and the upper limit may be, for example, 800 mM or less, 600 mM or less, 400 mM or less, 200 mM or less, 150 mM or less, 100 mM or less, 70 mM or less, 50 mM or less, or 40 mM or less. For example, the concentration may be 3 nM or more and 300 mM or less.

The composition for culture of cells according to this aspect of the present invention my be used to produce cell aggregates of pluripotent stem cells in high yields. The composition may also be used to produce cell aggregates having an appropriate size described above while maintaining the undifferentiated state of the stem cells.

Specific embodiments of the cell cycle arresting agent, the culture medium, and the pluripotent stem cell are as described above.

The step of producing a cell aggregate(s) of pluripotent stem cells from the cell culture composition comprises a step of subjecting the pluripotent stem cells to suspension culture in the composition for culture of cells as described above. Specific embodiments of this step are similar to the specific embodiments of the "step of culturing pluripotent stem cells in suspension in a culture medium comprising a pluripotent stem cell cycle arresting agent" in the method for suppressing aggregation of cells as described in the section <6. Methods for Suppressing Aggregation of Pluripotent Stem Cells>.

The composition for culture of cells may be prepared by adding a cell cycle arresting agent to a culture medium, then adding pluripotent stem cells, or by mixing pluripotent stem cells with a culture medium, then adding a cell cycle arresting agent. Preferably, the composition is prepared by adding a cell cycle arresting agent to a culture medium, then adding pluripotent stem cells. A stabilizer may also be added when adding the cell cycle arresting agent to the culture medium. The stabilizer is not particularly limited as long as it is a substance that contributes to: for example, stabilization of the cell cycle arresting agent in a liquid culture mediu; maintenance of its activity; prevention of adsorption to the culture vessel or the like, and does not inhibit suppression of the aggregation of pluripotent stem cells. The composition for culture of the cells may be prepared by freezing and storing a culture medium comprising a cell cycle arresting agent (optionally further comprising the stabilizer), and later thawing the culture medium and adding pluripotent stem cells thereto.

### <10. Pluripotent Stem Cell Culture Medium>

Another aspect of the present invention is a pluripotent stem cell culture medium comprising a culture medium and a cell cycle arresting agent, for example, at the concentration shown below, but not limited thereto,.

Regarding the concentration of the cell cycle arresting agent in the culture medium, the lower limit may be, for example, 3 ng/mL or more, 5 ng/mL or more, 7 ng/mL or more, 10 ng/mL or more, 20 ng/mL or more, 30 ng/mL or more, 40 ng/mL or more, 50 ng/mL or more, 100 ng/mL or more, 200 ng/mL or more, 300 ng/mL or more, 500 ng/mL or more L, 1 µg/mL or more, 5 µg/mL or more, 10 µg/mL or more, 15 µg/mL or more, 20 µg/mL or more, 50 µg/mL or more, 70 µg/mL or more, or 100 µg/mL or more, and the upper limit may be, for example, 50 mg/mL or less, 40 mg/mL or less, 35 mg/mL or less, 30 mg/mL or less, 25 mg/mL or less, 20 mg/mL or less, 15 mg/mL or less, or 10 mg/mL or less. For example, the concentration may be in a range of 3 ng/mL or more and 30 mg/mL or less. Alternatively, the lower limit may be, for example, 3 nM or more, 5 nM or more, 10 nM or more, 15 nM or more, 20 nM or more, 25 nM or more, 30 nM or more, 40 nM or more, 50 nM or more, 80 nM or more, 100 nM or more, 300 nM or more, 500 nM or more, 1 µM or more, 2 µM or more, 3 µM or more, 4 µM or more, 5 µM or more, 10 µM or more, 20 µM or more, 30 µM or more, 50 µM or more, 60 µM or more, 70 µM or more, 80 µM or more, or 100 µM or more, and the upper limit may be, for example, 800 mM or less, 600 mM or less, 400 mM or less, 200 mM or less, 150 mM or less, 100 mM or less, 70 mM or less, 50 mM or less, or 40 mM or less. For example, the concentration may be 3 nM or more and 300 mM or less.

According to this aspect of the present invention, the pluripotent stem cell culture medium may be used as a culture medium for producing cell aggregates from pluripotent stem cells by suspension culture in high yields. The cell culture medium may also be used to produce cell aggregates having an appropriate size as described above, while maintaining the undifferentiated state of the stem cells.

Specific embodiments of the cell cycle arresting agent, the culture medium, and the pluripotent stem cell are as described above.

An example of the step of producing a cell aggregate(s) of pluripotent stem cells from the pluripotent stem cell culture medium as described above includes the step of subjecting pluripotent stem cells to suspension culture in the pluripotent stem cell culture medium as described above. Specific embodiment of this step is similar to the specific embodiment of the "step of subjecting pluripotent stem cells to suspension culture in a culture medium comprising a cell cycle arresting agent" in the method for suppressing the aggregation of pluripotent stem cells as described in the section <6. Methods for Suppressing Aggregation of Pluripotent Stem Cells>.

According to one embodiment, the pluripotent stem cell culture medium may be frozen and stored until use, and thawed (melted) upon use.

### EXAMPLES

The present invention will be described in further detail with reference to the following examples, but the scope of the present invention is not limited to the examples.

### [Example 1] Maintenance culture of human ips cells

TkDN4-M cell lines (Institute of Medical Science, The University of Tokyo, Tokyo, Japan) were used as human iPS cells. Human iPS cells were seeded on cell culture dishes coated with Vitronectin (Thermo Fisher Scientific Co., Ltd.) and subjected to a maintenance culture using Essential 8™ (Thermo Fisher Scientific Co., Ltd.) as the culture medium. Accutase™ (Thermo Fisher Scientific Co., Ltd.) was used as a cell detachment agent during passages. In addition, only when the cells were seeded, Y-27632 (Wako Pure Chemical Industries, Ltd., Japan) at a concentration of 10 µM was added to a culture medium. The culture medium was exchanged every day. For experiments, human iPS cells (the number of passages was at most 50) were used.

### [Example 2] Confirmation of aggregation suppression effect by cell aggregation assay

The suppression effect on cell aggregation by adding a cell cycle arresting agent was examined.

### <Method>

Human iPS cells that had been cultured using the proceedures of Example 1 were treated with Accutase™ for 3 to 5 minutes and were detached and dispersed until becoming to single cells. The resulting cells were suspended in Essential 8™ culture medium containing a final concentration of 5 mg/mL of BSA (Wako Pure Chemical Industries, Ltd.), and a portion thereof was stained with trypan blue and the number of cells was counted. The cell suspension was prepared so as to contain 2 x 10⁵ cells per ml. To this cell suspension, Y27632 (Wako Pure Chemical Industries, Ltd.) was added to be a final concentration of 10 µM or, Y27632 at a final concentration of 10 µM is added. Separately, the concentrations of the following suppressors of cell aggregation were adjusted so that the final concentration of colchicine (Wako Pure Chemical Industries, Ltd., 039-03851) was 10 mg/mL (25 mM), the final concentration of demecolcine (Wako Pure Chemical Industries, Ltd., 049-16961) was 10 mg/mL (26.9 mM), the final concentration of cytochalasin D (Wako Pure Chemical Industries, Ltd., 037-17561) was 0.51 mg/mL (1 mM), the final concentration of cytochalasin B (Wako Pure Chemical Industries, Ltd., 030-17551) was 2.40 mg/mL (5 mM), the final concentration of vinblastine (Wako Pure Chemical Industries, Ltd., 037-17561) was 4.55 mg/mL (5 mM), the final concentration of griseofulvin (Wako Pure Chemical Industries, Ltd., 037-17561) was 17.6 mg/mL (50 mM), the concentration of DMSO (Wako Pure Chemical Industries, Ltd., 041-29351) was the stock concentration, the final concentration of latrunculin A (Wako Pure Chemical Industries, Ltd., 125-04363) was 0.42 mg/mL (1 mM), the final concentration of podophyllotoxin (Wako Pure Chemical Industries, Ltd., 161-20901) was 20.7 mg/mL (50 mM), the final concentration of vinorelbine (Wako Pure Chemical Industries, Ltd., 222-01641) was 5.4 mg/mL(5 mM), the final concentration of nocodazole (Wako Pure Chemical Industries, Ltd., 140-08531) was 5 mg/mL (16.7 mM), the final concentration of jasplakinolide (Cayman, 11705) was 0.14 mg/mL (0.2 mM), or the final concentration of docetaxel (Wako Pure Chemical Industries, Ltd., 047-31281) was 4.04 mg/mL (5 mM). Then the concentration-adjusted cell aggregation suppressor was added to the cell suspension so that the final concentration of colchicine was 10 ng/mL, the final concentration of demecolcine was 10 ng/mL, the final concentration of cytochalasin D was 20 nM, the final concentration of cytochalasin B was 1 µM, the final concentration of vinblastine was 2 µM, the final concentration of griseofulvin was 50 µM, the final concentration of DMSO was 0.3% (v/v), the final concentration of latrunculin A was 80 nM, the final concentration of podophyllotoxin was 80 nM, the final concentration of vinorelbine was 80 nM, the final concentration of nocodazole was 40 ng/mL, the final concentration of jasplakinolide was 16 nM, or the final concentration of docetaxel was 80 nM Then the cells were seeded at a rate of 1.3 ml/well in a 12-well plate for suspension culture (Sumitomo Bakelite Co., Ltd.). The cell-seeded plate was subjected to swirling culture on a rotary shaker (OPTIMA, Inc.) at a speed of 90 rpm along the horizontal plane to draw a circle with a swirling width (diameter) of 25 mm, and the cells were subjected to suspension culture under the environment of 5%CO₂ and 37°C. At the next day after the start of culture (Day 1 of culture), images were obtained by phase contrast microscopy.

### <Results>

Figs. 1A, 1B and 1C show the micrographs after the suspension culture described above (Day 1 of culture). As a result of the observation, larger cell aggregates of 1 mm or more were formed in the condition in which only Y27632 was added (control), whereas the cell aggregates of 500 µm or less in diameter were formed in the conditions in which the cell cycle arresting agent was added.

### [Example 3]

### Effect of the presence of cell cycle arresting agent on cell proliferation ability and undifferentiation ability after formation of aggregate

Suspension culture of human iPS cells was performed, and the glucose consumption, the cell yield, and the percentage of cells positive for undifferentiation markers were determined to analyze the effect of cell cycle arresting agents on the cells.

### <Method>

Cell suspensions were prepared in the same manner as in Example 2, and separately, the concentrations of the suppressors of cell aggregation were adjusted so that the final concentration of demecolcine (same as above) was 10 mg/mL (26.9 mM), the final concentration of cytochalasin D (same as above) was 0.51 mg/mL (1 mM), the concentration of DMSO (the same as above) was the stock concentration, or the final concentration of jasplakinolide (Cayman, 11705) was 0.14 mg/mL (0.2 mM). Then the concentration-adjusted cell aggregation suppressors were added to the cell suspensions so that the final concentration of demecolcine was 10 ng/mL, the final concentration of cytochalasin D was 20 nM, the final concentration of DMSO was 1%(v/v), or the final concentration of jasplakinolide was 20 nM, and the cells were seeded at a rate of 4 mL/well in a 6-well plate for suspension culture (Sumitomo Bakelite Co., Ltd., Japan). The cell-seeded plate was subjected to a swirling culture on a rotary shaker (OPTIMA, Inc., Japan) at a speed of 90 rpm along the horizontal plane to draw a circle with a swirling width (diameter) of 25 mm and cells were subjected to suspension culture under the environment of 5% CO₂ and 37°C. After the next day of culture (Day 1 of culture), the culture medium was exchanged daily with a fresh culture medium (Essential 8™ culture medium containing BSA (Wako Pure Chemical Industries, Ltd.) at a final concentration of 5 mg/mL), and the culture was continued until 5 days after the start of culture. Images were obtained by phase contrast microscopy every day during culture. While 182 to 252 cell aggregates in images obtained on Day 1 of culture being observed and compared using a micrograph scale, the width (referred to as "ϕ") of the widest portion of each cell aggregate was measured, distribution thereof was examined, and average ± standard deviation was calculated. The concentration of glucose contained in the culture supernatant collected at the time of culture medium exchange was measured with biosensor BF-5iD (Prince Measuring Equipment Co., Ltd.) to calculate glucose consumption.

In addition, on Day 5 of culture, cell aggregates were collected, dispersed with Accutase™, and then suspended in Essential 8™ culture medium containing 5 mg/mL BSA. A portion of this cell suspension was stained with trypan blue and the number of the cells was counted. After the above-mentioned cell suspensions were centrifuged at 300 g for 3 minutes, the supernatant was then removed, and the cells were washed with PBS (phosphate buffered saline). Next, the cells were fixed with 4% paraformaldehyde (Wako Pure Chemical Industries, Ltd.) at room temperature for 20 minutes, then washed 3 times with PBS. After the cells were resuspended in 300 µL of PBS, 3 mL of cold methanol was added while stirring using voltex, and subjected to treatment for permeabilization at -20°C overnight or more. After 3 washes with 3% FBS (fetal bovine serum)/PBS, the cells were resuspended in 3% FBS (fetal bovine serum)/PBS and blocked at room temperature for 30 minutes to 1 hour. Subsequently, the cells were stained with fluorescently labeled anti-SOX2 antibody (Cat. No. 656110, Biolegend, Inc.) and fluorescently labeled anti-OCT4 antibody (Cat. No. 653703, Biolegend, Inc.) and fluorescently labeled anti-Nanog antibody (Cat. No. 674010, Biolegend, Inc.) at 4°C for 30 minutes to 1 hour. After washed once with 3% FBS (fetal calf serum)/PBS, the cells were made to pass through a cell strainer. The resulting cells were analyzed on FACSVerse™ flow cytometer (BD Biosciences).

Furthermore, cell yields were also measured on Day 5 of culture. The following procedures were used to measure the cell yields. Specifically, the formed cell aggregates were treated with Accutase™ for 5 to 10 minutes, pipetted using a blue tip to monodisperse the cells, and stained with trypan blue. After that, the number of cells was counted using a hemocytometer to determine the cell yield.

### <Results

Figs. 2A to 2E are the micrographs observed from Day 1 to Day 5 of culture. In Figs. 2B to 2E, compared to Fig. 2A (control), cell aggregates were formed after seeding to Day 1 of culture, and, by continuing the culture, the cells were gradually proliferated and the cell aggregates became larger and almost uniform and were eventually an appropriate size.

Figs. 6A to 6D are the distribution diagrams of the cell aggregate size (the diameter of cell aggregate, or the size of the widest portion of the cell aggregate) on Day 1 of culture. Tables 1 to 4 show the number and percentage of cell aggregates by size on Day 1 of culture. The results of the calculated average ± standard deviation of cell aggregate sizes (diameters) on Day 1 of culture under each culture condition were: 132.5 ± 34.8 µm for addition of cytochalasin D; 162.7 ± 37.2 µm for addition of jasplakinolide; 184.9 ± 44.9 µm for addition of demecolcine, and 164.8 ± 31.8 µm for addition of DMSO.

**[Table 1]**

| Number and percentage of cell aggregates by size (addition of Cytochalasin D) | | | | | | | | | | | | | | |
|---|---|---|---|---|---|---|---|---|---|---|---|---|---|---|
| Size range (µm) | 40- | 60- | 80- | 100- | 120- | 140- | 160- | 180- | 200- | 220- | 240- | 260- | 280- | 300- |
| Number | 10 | 20 | 19 | 26 | 60 | 66 | 40 | 10 | 0 | 1 | 0 | 0 | 0 | 0 |
| Percentage | 4.0% | 7.9% | 7.5% | 10.3% | 23.8% | 26.2% | 15.9% | 4.0% | 0.0% | 0.4% | 0.0% | 0.0% | 0.0% | 0.0% |

In case of the addition of cytochalasin D, the percentage of cell aggregates whose cell aggregate size (diameter) was 40 µm or more and 300 µm or less was 100% relative to the total cell aggregate count. The percentage of cell aggregates whose cell aggregate size (diameter) was 60 µm or more and 300 µm or less was 96%. The percentage of cell aggregates whose cell aggregate size (diameter) was 80 µm or more and 300 µm or less was 88.1%. The percentage of cell aggregates whose cell aggregate size (diameter) was 100 µm or more and 300 µm or less was 80.6%.

**[Table 2]**

| Number and percentage of cell aggregates by size (addition of Jasplakinolide) | | | | | | | | | | | | | | |
|---|---|---|---|---|---|---|---|---|---|---|---|---|---|---|
| Size range (µm) | 40- | 60- | 80- | 100- | 120- | 140- | 160- | 180- | 200- | 220- | 240- | 260- | 280- | 300- |
| Number | 4 | 4 | 1 | 8 | 23 | 52 | 66 | 30 | 9 | 4 | 1 | 1 | 2 | 1 |
| Percentage | 1.9% | 1.9% | 0.5% | 3.9% | 11.2% | 25.2% | 32.0% | 14.6% | 4.4% | 1.9% | 0.5% | 0.5% | 1.0% | 0.5% |

In case of the addition of jasplakinolide, the percentage of cell aggregates whose cell aggregate size (diameter) was 40 µm or more and 300 µm or less was 100% relative to the total cell aggregate count. The percentage of cell aggregates whose cell aggregate size (diameter) was 60 µm or more and 300 µm or less was 98.1%. The percentage of cell aggregates whose cell aggregate size (diameter) was 80 µm or more and 300 µm or less was 96.2%. The percentage of cell aggregates whose cell aggregate size (diameter) was 100 µm or more and 300 µm or less was 95.7%.

**[Table 3]**

| Number and percentage of cell aggregates by size (addition of Demecolcine) | | | | | | | | | | | | | | |
|---|---|---|---|---|---|---|---|---|---|---|---|---|---|---|
| Size range (µm) | 40- | 60- | 80- | 100- | 120- | 140- | 160- | 180- | 200- | 220- | 240- | 260- | 280- | 300- |
| Number | 5 | 7 | 2 | 0 | 2 | 16 | 37 | 45 | 38 | 19 | 8 | 2 | 0 | 1 |
| Percentage | 2.7% | 3.8% | 1.1% | 0.0% | 1.1% | 8.8% | 20.3% | 24.7% | 20.9% | 10.4% | 4.4% | 1.1% | 0.0% | 0.5% |

In case of the addition of demecolcine, the percentage of cell aggregates whose cell aggregate size (diameter) was 40 µm or more and 300 µm or less was 100% relative to the total cell aggregate count. The percentage of cell aggregates whose cell aggregate size (diameter) was 60 µm or more and 300 µm or less was 97.3%. The percentage of cell aggregates whose cell aggregate size (diameter) was 80 µm or more and 300 µm or less was 93.5%. The percentage of cell aggregates whose cell aggregate size (diameter) was 100 µm or more and 300 µm or less was 92.4%.

**[Table 4]**

| Number and percentage of cell aggregates by size (addition of DMSO) | | | | | | | | | | | | | | |
|---|---|---|---|---|---|---|---|---|---|---|---|---|---|---|
| Size range (µm) | 40- | 60- | 80- | 100- | 120- | 140- | 160- | 180- | 200- | 220- | 240- | 260- | 280- | 300- |
| Number | 0 | 3 | 5 | 2 | 24 | 56 | 56 | 33 | 12 | 5 | 1 | 1 | 1 | 0 |
| Percentage | 0.0% | 1.5% | 2.5% | 1.0% | 12.1% | 28.1% | 28.1% | 16.6% | 6.0% | 2.5% | 0.5% | 0.5% | 0.5% | 0.0% |

In case of the addition of DMSO, the percentage of cell aggregates whose size (diameter) was 40 µm or more and 300 µm or less was 100% relative to the total cell aggregate count. The percentage of cell aggregates whose cell aggregate size (diameter) was 60 µm or more and 300 µm or less was 100%. The percentage of cell aggregates whose size (diameter) was 80 µm or more and 300 µm or less was 98.5%. The percentage of cell aggregates whose cell aggregate size (diameter) was 100 µm or more and 300 µm or less was 96.0%.

Glucose consumption is shown in Fig. 3, and cell yields on Day 5 of culture are shown in Fig. 4, respectively. The glucose consumption under the condition that the cell cycle arresting agent was added was greater than the glucose consumption under the condition that the cell cycle arresting agent was absent, suggesting that the cells proliferated. In fact, it was revealed that the cell yiel increased 5-fold or more than the seeded cell number, on Day 5 of culture.

Figs. 5A to 5D show the results of the measured percentages of cells positive for undifferentiation markers. In case of the cells obtained by producing cell aggregates under each condition followed by proliferation in suspension culture, the percentages of cells positive for the markers SOX2, OCT4 and Nanog were found to be 99% or more, 97% or more, and 99% or more, respectively. As a result, it was confirmed that the human iPS cell aggregates, which had been formed by the addition of the cell cycle arresting agents, maintained the undifferentiated state of the iPS cells.

### INDUSTRIAL APPLICABILITY

According to the present invention, it is possible in suspension culture of pluripotent stem cells to produce cell aggregates with both of high cell viability and appropriate size (500 µm or less) in high yields, while maintaining the undifferentiated state of the cells and suppressing the formation of cell aggregates with the size greater than 1000 µm (1 mm). Because pluripotent stem cells like ES cells and iPS cells are capable of inducing their differentiation into various cells in the living body, the present invention makes it possible to supply such cells for regenerative medicine.

### Sequence Listing Free Text

SEQ ID NO: 1: Synthetic peptide
SEQ ID NO: 2: Synthetic peptide
SEQ ID NO: 3: Synthetic peptide
SEQ ID NO: 4: Synthetic peptide

All the publications, patents, and patent applications cited herein are incorporated herein by reference in their entirety.

## Claims

1. A suppressor of pluripotent stem cell aggregation for use in suspension culture of pluripotent stem cells, comprising a cell cycle arresting agent.

2. The suppressor of pluripotent stem cell aggregation according to claim 1, wherein a concentration of the cell cycle arresting agent is 55.0 mg/mL or more and 1.1 g/mL or less.

3. The suppressor of pluripotent stem cell aggregation according to claim 1 or 2, wherein the cell cycle arresting agent is at least one substance selected from G1 phase-arresting agents, G1/S phase-arresting agents, G2 phase-arresting agents, G2/M phase-arresting agents, and M phase-arresting agents.

4. The suppressor of pluripotent stem cell aggregation according to any one of claims 1 to 3, wherein the cell cycle arresting agent is at least one substance selected from the group consisting of colchicine, dimethylsulfoxide (DMSO), cytochalasin D, cytochalasin B, docetaxel, podophyllotoxin, demecolcine, nocodazole, jasplakinolide, vinblastine, griseofulvin, vinorelbine, latorunculin A, and salts thereof.

5. The suppressor of pluripotent stem cell aggregation according to claim 3, wherein the G1 phase-arresting agent is at least one substance selected from colchicine and dimethylsulfoxide (DMSO).

6. The suppressor of pluripotent stem cell according to claim 3, wherein the G1/S phase-arresting agent is cytochalasin D.

7. The suppressor of pluripotent stem cell aggregation according to claim 3, wherein the G2 phase-arresting agent is at least one substance selected from cytochalasin D, colchicine, and docetaxel.

8. The suppressor of pluripotent stem cell aggregation according to claim 3, wherein the G2/M phase-arresting agent is at least one substance selected from podophyllotoxin, demecolcine, latorunculin A, cytochalasin B, and nocodazole.

9. The suppressor of pluripotent stem cell aggregation according to claim 3, wherein the M phase-arresting agent is jasplakinolide.

10. A method for producing a pluripotent stem cell aggregate(s), comprising a step of subjecting pluripotent stem cells to suspension culture in a culture medium comprising a cell cycle arresting agent.

11. The method according to claim 10, wherein a concentration of the cell cycle arresting agent is 3 ng/mL or more and 30 mg/mL or less in the culture medium.

12. The method according to claim 10 or 11, wherein the cell cycle arresting agent is at least one substance selected from G1 phase-arresting agenst, G1/S phase-arresting agents, G2 phase-arresting agents, G2/M phase-arresting agents, and M phase-arresting agents.

13. The method according to any one of claims 10 to 12, wherein the cell cycle arresting agent is at least one substance selected from the group consisting of colchicine, dimethylsulfoxide (DMSO), cytochalasin D, cytochalasin B, docetaxel, podophyllotoxin, demecolcine, nocodazole, jasplakinolide, vinblastine, griseofulvin, vinorelbine, latorunculin A, and salts thereof.

14. The method according to claim 12, wherein the G1 phase-arresting agent is at least one substance selected from colchicine and dimethylsulfoxide (DMSO).

15. The method according to claim 12, wherein the G1/S phase-arresting agent is cytochalasin D.

16. The method according to claim 12, wherein the G2 phase-arresting agent is at least one substance selected from cytochalasin D, colchicine, and docetaxel.

17. The method according to claim 12, wherein the G2/M phase-arresting agent is at least one substance selected from podophyllotoxin, demecolcine, latorunculin A, cytochalasin B, and nocodazole.

18. The method according to claim 12, wherein the M phase-arresting agent is jasplakinolide.

19. A pluripotent stem cell aggregate obtained by the method according to any one of claims 10 to 18.

20. A composition for culture of pluripotent stem cells, comprising a cell aggregate(s) of pluripotent stem cells, a culture medium, and a cell cycle arresting agent, wherein a concentration of the cell cycle arresting agent is 3 ng/mL or more and 30 mg/mL or less.

21. The composition for culture of pluripotent stem cells according to claim 20, wherein the cell cycle arresting agent is at least one substance selected from G1 phase-arresting agents, G1/S phase-arresting agents, G2 phase-arresting agents, G2/M phase-arresting agents, and M phase-arresting agents.

22. The composition for culture of pluripotent stem cells according to claim 20 or 21, wherein the cell cycle arresting agent is at least one substance selected from the group consisting of colchicine, dimethylsulfoxide (DMSO), cytochalasin D, cytochalasin B, docetaxel, podophyllotoxin, demecolcine, nocodazole, jasplakinolide, vinblastine, griseofulvin, vinorelbine, latorunculin A, and salts thereof.

23. The composition for culture of pluripotent stem cells according to any one of claims 20 to 22, further comprising a growth factor.
